# EUROPEAN PATENT SPECIFICATION

(11) **EP 3 445 847 B1**
(45) Date of publication and mention of the grant of the patent: **05.06.2024**
(21) Application number: 17724147.8
(22) Date of filing: 20.04.2017
(51) Int. Cl.: C12N 5/0783

(54) **IMMUNOMODULATORY IL2R FUSION PROTEINS AND USES THEREOF**
IMMUNOMODULATORISCHE IL2R-FUSIONSPROTEINE UND VERWENDUNGEN DAVON
PROTÉINES DE FUSION IL2R IMMUNOMODULATRICES ET LEURS UTILISATIONS

(30) Priority: 20.04.2016 US 201662325428 P
(43) Date of publication of application: 27.02.2019
(73) Proprietor: Fred Hutchinson Cancer Center, Seattle, WA 98109 (US)
(72) Inventor: SCHMITT, Thomas, M., Seattle, WA 98119 (US); GREENBERG, Philip, D., Mercer Island, WA 98040 (US); STROMNES, Ingunn, M., Kenmore, WA 98028 (US)
(74) Representative: Hoffmann Eitle
(86) International application number: PCT/US2017/028693
(87) International publication number: WO 2017/184901

(56) References cited:
- WO-A1-94/22914
- LAWRENCE S. EVANS ET AL: "Expression of Chimeric Granulocyte-Macrophage Colony-Stimulating Factor/Interleukin 2 Receptors in Human Cytotoxic T Lymphocyte Clones Results in Granulocyte-Macrophage Colony-Stimulating Factor-Dependent Growth", HUMAN GENE THERAPY, vol. 10, no. 12, 10 August 1999 (1999-08-10), pages 1941-1951, XP055382781, US ISSN: 1043-0342, DOI: 10.1089/10430349950017301
- INGUNN?M. STROMNES ET AL: "T Cells Engineered against a Native Antigen Can Surmount Immunologic and Physical Barriers to Treat Pancreatic Ductal Adenocarcinoma", CANCER CELL, vol. 28, no. 5, 1 November 2015 (2015-11-01), pages 638-652, XP055382318, US ISSN: 1535-6108, DOI: 10.1016/j.ccell.2015.09.022
- GREENBERG PHILIP D ET AL: "Employing TCRs in engineered T cells to develop therapeutic reagents for effectively targeting malignancies", CANCER RESEARCH, vol. 75, no. Suppl. 15, August 2015 (2015-08), pages SY31-03, XP002771214, & 106TH ANNUAL MEETING OF THE AMERICAN-ASSOCIATION-FOR-CANCER-RESEARCH (AACR); PHILADELPHIA, PA, USA; APRIL 18 -22, 2015
- STROMNES INGUNN M ET AL: "Engineered T cell-receptor based therapy of pancreatic ductal adenocarcinoma", CANCER IMMUNOLOGY RESEARCH, vol. 4, no. 1, Suppl. S, January 2016 (2016-01), page IA38, XP002771215, & CRI-CIMT-EATI-AACR INAUGURAL INTERNATIONAL CANCER IMMUNOTHERAPY CONFERENCE; NEW YORK, NY, USA; SEPTEMBER 16 -19, 2015
- T. M. SCHMITT ET AL: "Enhanced-affinity murine T-cell receptors for tumor/self-antigens can be safe in gene therapy despite surpassing the threshold for thymic selection", BLOOD, vol. 122, no. 3, 18 July 2013 (2013-07-18) , pages 348-356, XP055383506, US ISSN: 0006-4971, DOI: 10.1182/blood-2013-01-478164

## Description

### BACKGROUND

T cell-based immunotherapies began to be developed when tumor-reactive T cells were found among a population of tumor-infiltrating lymphocytes (TILs) (Clark et al., Cancer Res. 29:705, 1969). One strategy, known as adoptive T cell transfer, in some contexts involves the isolation of tumor infiltrating lymphocytes pre-selected for tumor-reactivity, clonal expansion of the tumor-reactive T cells induced by anti-CD3 and anti-CD28 antibodies in the presence of IL-2, and finally infusing the expanded cell population back to the tumor-bearing patient (together with chemotherapy and repetitive administration of IL-2) (Dudley et al., Science 298:850, 2002). This form of adoptive T cell therapy with tumor infiltrating lymphocytes can be technically cumbersome and leads to complete remission in only a minor fraction of patients with melanoma and is rarely effective in other cancers (Besser et al., Clin. Cancer Res. 16:2646, 2010).

Isolation of tumor-reactive T cell clones led to the development of another immunotherapeutic approach - the generation of recombinant T cell receptors (TCRs) specific for particular antigens, which may be introduced into T cells, *e.g.,* using a vector delivery system, to confer specificity for a desired target such as a tumor-associated peptide presented by a major histocompatibility complex (MHC) molecule expressed on a tumor cell (known as human leukocyte antigen (HLA) molecule in humans). Another approach introduces a synthetic receptor, termed a chimeric antigen receptor (CAR), which generally contains an antigen-binding domain, which, e.g., in the context of anti-tumor therapy can bind to a tumor-specific or associated antigen, linked to one or more intracellular component comprising an effector domains, such as a primary signaling domain such as a TCR signaling domain or in some contexts costimulatory signaling domains. Unlike administration of TILs, the basic procedure for engineered TCR or CAR T cell immunotherapy is generally to genetically modify human T cells with a transgene encoding a tumor targeting moiety, *ex vivo* expansion of the recombinant T cells, and transfusing the expanded recombinant T cells back into patients.

Adoptive T cell therapy using T cells expressing recombinant TCRs has been shown to have a promising clinical benefit, especially in certain B cell cancers. However, effective T cell activation often requires or is enhanced by a concurrent co-stimulatory signal (Chen and Flies, Nat. Rev. Immunol. 13: 227-242, 2013). In the tumor microenvironment, co-stimulatory molecules are generally downregulated. As a result, exogenous stimulus via IL-2 is typically needed for T cells that express recombinant TCRs specific for cancer antigens.

Activation of T cells is initiated when the TCR engages a specific peptide presented in MHC on an antigen-presenting cell (APC) (Rossy et al., Frontiers in Immunol. 3:1, 2012). Multiple cytokines, including IL-2, can affect T cell proliferation and survival. The magnitude of the T cell response is regulated in part by signals delivered to T cells through cytokine receptors. The IL-2 receptor (IL-2R) complex is a heterotrimer comprised of a unique α chain (CD25), a β chain shared with the IL-15 receptor, and a γ chain shared with the IL-4, IL-7, IL-9, and IL-15 receptors, all of which also can deliver proliferative signals (Nelson and Willerford, Adv. Immunol. 70:1, 1998).

CD8⁺ T cells generally lose the ability to produce IL-2 after differentiation into effector T cells (CTLs) (Aruga et al., J. Leukocyte Biol. 61:507, 1997). Differentiated effector CD8⁺ T cells also retain the capacity to secrete many cytokines, including granulocyte-macrophage colony-stimulating factor (GM-CSF) (Aruga *et al.,* 1997), but do not express the GM-CSF receptor. Administration of exogenous IL-2 systemically can be used to prolong CTL longevity *in vivo* (Cheever and Chen, Immuno. Rev. 157:177, 1997), but IL-2 treatment has been associated with severe toxicity (Dalgleish, Gene Ther. 1:83, 1994).

Evans et al., Human Gene Therapy 10:1941-1951 (1999) as well as WO9422914 describe the transfection of human CTLs with a chimeric GM-CSF/IL2 receptor, resulting in an autocrine growth loop.

There remains a need in the immunotherapy field for augmented signaling and effectiveness by means of the IL-2R in responding CD8⁺ T cells *in vivo.* Presently disclosed embodiments address these needs and provide other related advantages.

### SUMMARY OF THE INVENTION

The present invention provides a host cell, comprising a fusion protein and an antigen binding protein, wherein the fusion protein comprises a transmembrane domain disposed between an extracellular component comprising a cytokine binding domain or a cytokine binding domain portion thereof, and an intracellular component comprising an IL-2R intracellular portion, intracellular signaling domain or an intracellular signaling domain portion thereof, and wherein the antigen binding protein is a T cell receptor (TCR), a chimeric antigen receptor (CAR), or both, wherein the TCR is exogenous to the host cell; and wherein the antigen binding protein is specific for a cancer-specific antigen.

The present invention also provides a host cell comprising a nucleic acid molecule encoding the fusion protein and the antigen binding protein disclosed in the previous paragraph.

The present invention also provides a composition comprising the host cell of the present invention and a pharmaceutically acceptable carrier, diluent, or excipient.

The present invention provides the host cell of the present invention or composition of the present invention for use in treating cancer.

Further aspects of the invention are provided in the claims.

Any disclosure related to a method of treatment in the present patent should be construed as referring to the host cell of the present invention or composition of the present invention for use in said method of treatment in accordance with Article 54(5) EPC.

### BRIEF DESCRIPTION OF THE DRAWINGS

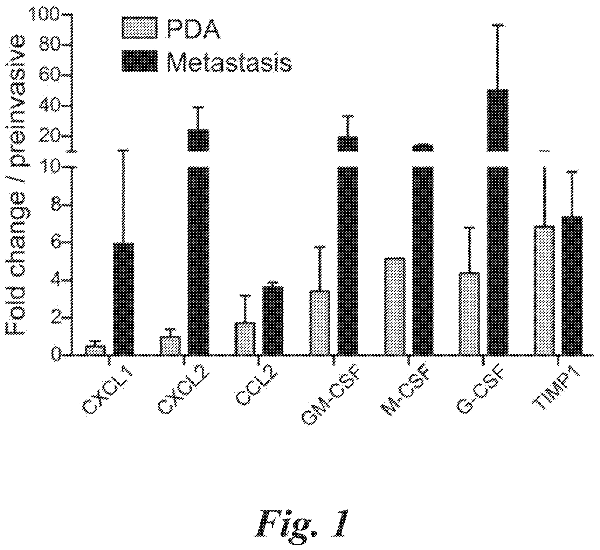

Figure 1 shows the relative gene expression of select cytokines and chemokines as determined by quantitative PCR (data represent mean ± SEM of 3 independently derived primary invasive tumor (pancreatic ductal adenocarcinoma, PDA) and paired metastatic cell preparations and are normalized to expression in preinvasive cells.

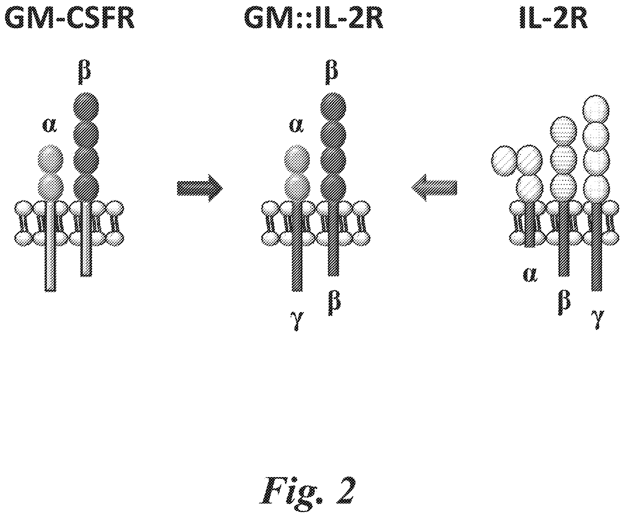

Figure 2 is an illustration of exemplary fusion proteins of this disclosure. A first fusion protein comprises an extracellular component from CSF2Rα and an intracellular component from IL-2Ry, and a second fusion protein comprises an extracellular component from CSF2Rβ and an intracellular component from IL-2Rβ. The illustration shows the fusion proteins located in a cell membrane (e.g., T cell membrane) and forming a complex, which is a heterodimer.

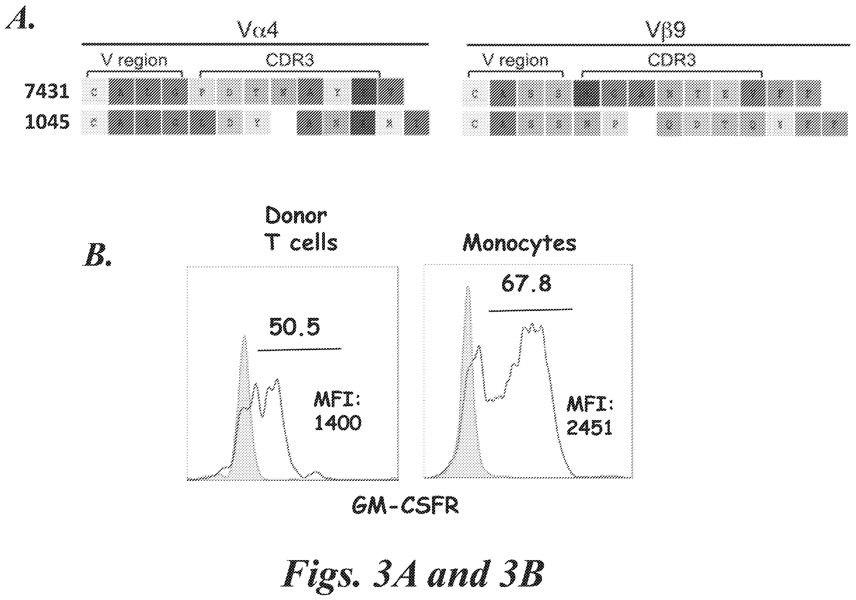

Figures 3A and 3B show (A) CDR3 sequences of Vα4 and Vβ9 chains cloned from the highest avidity MSLN₄₀₆₋₄₁₄-specific T cell clones isolated from wild-type and Msln^{-/-} mice; and (B) results of flow cytometric assessment of the percentage of donor (gating on Thy1.1+/Vβ9+ (indicative of mesothelin-specific TCR_{I1045}⁺)) CD8+ T cells in the blood of animals, following adoptive transfer, that expressed a GM-CSFR extracellular motif (left panel). The detection of the motif on the T cells indicated the expression of the GM-CSF::IL-2R fusion protein. Staining of monocytes (right panel), which express GM-CSFR, served as a positive control.

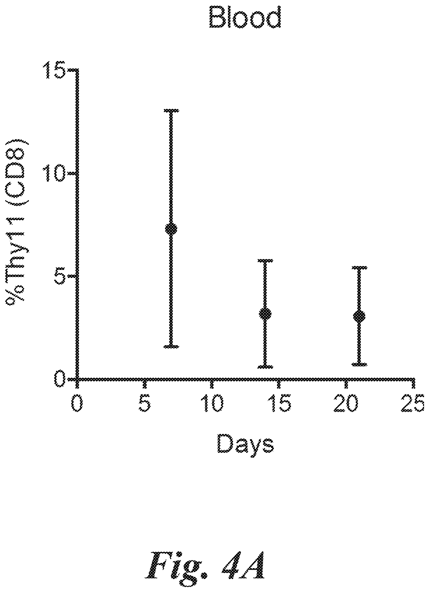

Figures 4A to 4C show (A) the overall percentage of donor (gating on Thy1.1+, CD8+) T cells detected in the blood over time following adoptive transfer (days 0, 5, 10, 15, 20, 25); (B) the percentage of the donor (gating on Thy1.1+/Vβ9+) CD8+ T cells in the blood at day 0 and day 14 (left and right panels, respectively) in which the GM-CSFR extracellular motif was detected, indicating the expression of the GM-CSF::IL-2R fusion protein; (C) shows the relative percentage of donor cells in the blood represented by cells expressing the GM-CSF::IL-2R fusion protein ("GM/IL2R TCR₁₀₄₅") as compared to those in which the fusion protein was not detected ("TCR₁₀₄₅"), indicating that the fusion protein provided a survival and/or expansion advantage to the mesothelin-targeting TCR₁₀₄₅-expressing T cells in this study.

### DETAILED DESCRIPTION

The instant disclosure provides fusion proteins that modulate signaling in a host cell, such as an immune cell. For example, fusion proteins of this disclosure can provide an activation or proliferation signal to a human T cell, wherein the T cell may optionally be engineered to have a preferred cancer antigen-specific T cell receptor (TCR) or chimeric antigen receptor (CAR) or both. For example, these fusion proteins can interact with a cytokine or chemokine of interest to provide T cells, such as T cells containing an antigen-specific TCR or CAR, a survival and/or expansion advantage, which is consistent with utility of the construct to improve persistence and exposure to transferred cells, including improving efficacy in a tumor microenvironment.

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, the term "about" means ± 20% of the indicated range, value, or structure, unless otherwise indicated. It should be understood that the terms "a" and "an" as used herein refer to "one or more" of the enumerated components. The use of the alternative (*e.g*., "or") should be understood to mean either one, both, or any combination thereof of the alternatives. As used herein, the terms "include," "have" and "comprise" are used synonymously, which terms and variants thereof are intended to be construed as non-limiting.

The term "consisting essentially of" limits the scope of a claim to the specified materials or steps, or to those that do not materially affect the basic characteristics of a claimed invention. For example, a protein domain, region, or module (*e.g*., a binding domain, hinge region, linker module) or a protein (which may have one or more domains, regions, or modules) "consists essentially of" a particular amino acid sequence when the amino acid sequence of a domain, region, or module or protein includes extensions, deletions, mutations, or any combination thereof (*e.g*., amino acids at the amino- or carboxy-terminus or between domains) that, in combination, contribute to at most 20% (e.g., at most 15%, 10%, 8%, 6%, 5%, 4%, 3%, 2%, or 1%) of the length of a domain, region, or module or protein and do not substantially affect (*i.e.,* do not reduce the activity by more than 50%, such as no more than 40%, 30%, 25%, 20%, 15%, 10%, 5%, or 1%) the activity of the domain(s), region(s), module(s), or protein (e.g., the target binding affinity of a binding protein).

As used herein, "heterologous" or "non-endogenous" or "exogenous" refers to any gene, protein, compound, molecule, or activity that is not native to a host cell or a subject, or is any gene, protein, compound, molecule, or activity native to a host or host cell that has been altered or mutated such that the structure, activity or both is different as between the native and mutated molecules. In certain embodiments, heterologous, non-endogenous or exogenous molecules (*e.g*., receptors, ligands) may not be endogenous to a host cell or subject, but instead nucleic acids encoding such molecules may have been added to a host cell by conjugation, transformation, transfection, electroporation, or the like, wherein the added nucleic acid molecule may integrate into a host cell genome or can exist as extra-chromosomal genetic material (*e.g*., as a plasmid or other self-replicating vector). The term "homologous" or "homolog" refers to a molecule or activity found in or derived from a host cell, species, or strain. For example, a heterologous or exogenous molecule or gene encoding the molecule may be homologous to a native host or host cell molecule or gene that encodes the molecule, respectively, but may have an altered structure, sequence, expression level or combinations thereof. A non-endogenous molecule may be from the same species, a different species, or a combination thereof.

As used herein, the term "endogenous" or "native" refers to a gene, protein, compound, molecule, or activity that is normally present in a host or host cell and has no engineered alterations.

A "binding domain" (also referred to as a "binding region" or "binding moiety"), as used herein, refers to a molecule, such as a peptide, oligopeptide, polypeptide or protein, that possesses the ability to specifically and non-covalently associate, unite, or combine with a target molecule. A binding domain includes any naturally occurring, synthetic, semi-synthetic, or recombinantly produced binding partner for a biological molecule or other target of interest or binding protein thereof. In some embodiments, the binding domain is an antigen-binding domain from, for example, an antibody or T cell receptor (TCR) or comprises a functional binding domain or antigen-binding fragment thereof (*e.g*., domain antibodies, sFv, scFv, Fab, single chain TCRs (scTCRs), or the like). In other embodiments, a binding domain or binding portions thereof binds to a cytokine or chemokine, such as GM-CSF.

In some embodiments, "specifically binds" refers to an association or union of a binding domain, or a fusion protein thereof, to a target molecule with an affinity or Kₐ (*i.e.,* an equilibrium association constant of a particular binding interaction with units of 1/M) equal to or greater than 10⁵ M⁻¹, or binds to such target molecule while not significantly associating or uniting with any other molecules or components in a sample. Binding domains (or fusion proteins thereof) may be classified as "high affinity" binding domains (or fusion proteins thereof) or "low affinity" binding domains (or fusion proteins thereof). "High affinity" binding domains refer to those binding domains with a Kₐ of at least 10⁷ M⁻¹, at least 10⁸ M⁻¹, at least 10⁹ M⁻¹, at least 10¹⁰ M⁻¹, at least 10¹¹ M⁻¹, at least 10¹² M⁻¹, or at least 10¹³ M⁻¹. "Low affinity" binding domains refer to those binding domains with a Kₐ of up to 10⁷ M⁻¹, up to 10⁶ M⁻¹, up to 10⁵ M⁻¹. Alternatively, affinity may be defined as an equilibrium dissociation constant (K_{d}) of a particular binding interaction with units of M (*e.g.,* 10⁻⁵ M to 10⁻¹³ M). In certain embodiments, a binding domain may have "enhanced affinity," which refers to a selected or engineered binding domain with stronger binding to a target antigen than a wild type (or parent) binding domain. For example, enhanced affinity may be due to a Ka (equilibrium association constant) for the target antigen that is higher than the wild type binding domain, or due to a K_{d} (dissociation constant) for the target antigen that is less than that of the wild type binding domain, or due to an off-rate (K_{off}) for the target antigen that is less than that of the wild type binding domain. A variety of assays are known for identifying binding domains of the present disclosure that specifically bind a particular target, as well as determining binding domain or fusion protein affinities, such as Western blot, ELISA, and Biacore^{®} analysis (*see also, e.g.,* Scatchard et al., Ann. N.Y. Acad. Sci. 51:660, 1949; and U.S. Patent Nos. 5,283,173, 5,468,614, or the equivalent).

As used herein, a "fusion protein" refers to a polypeptide that, in a single chain, has at least two distinct domains, wherein the domains are not naturally found together in a protein. A nucleic acid molecule encoding a fusion protein may be constructed using PCR, recombinantly engineered, or the like, or such fusion proteins can be made using methods of protein synthesis. A fusion protein may further contain other components (*e.g.*, covalently bound), such as a tag or bioactive molecule. In certain embodiments, a fusion protein expressed or produced by a host cell (*e.g.,* T cell) locates to the cell surface, where the fusion protein is anchored to the cell membrane with a portion of the fusion protein located extracellularly (*e.g*., containing a binding domain) and a portion of the fusion protein located intracellularly (*e.g*., containing a signaling domain).

As used herein, an "extracellular component" refers to a portion or domain of a fusion protein that is located outside of a cell and that is capable of specifically interacting or associating with another molecule or compound to induce a biological effect by transmitting a signal to the intracellular component of the fusion protein. For example, a cytokine binding domain is capable of associating with a specific cytokine and inducing signal transduction into the cell via the intracellular component of the fusion protein.

As used herein, an "intracellular component" refers to a portion or domain of a fusion protein that is located in the cytoplasm of a host cell and that is capable of transmitting signals to the cell via an "intracellular signaling domain" by interacting with a signaling molecule or with another intracellular component.

As used herein, an "intracellular signaling domain" is an intracellular portion of molecule, such as one used in a fusion protein of this disclosure, that can directly or indirectly promote a response, such as a co-stimulatory, positive, or activating biological or physiological response in a cell when receiving the appropriate signal. In certain embodiments, an intracellular signaling domain is part of a protein or protein complex that receives a signal when bound, or itself can bind directly to a target molecule to transmit a signal to other components in the cell. An intracellular signaling domain may directly promote a cellular response when it contains one or more signaling domains or motifs, such as a Box 1 motif (*e.g*., JAK interacting domain found on a common gamma chain), a kinase domain, an immunoreceptor tyrosine-based activation motif (ITAM), a co-stimulatory domain, or the like. In other embodiments, an intracellular signaling domain will indirectly promote a cellular response by associating with one or more other proteins that in turn directly promote a cellular response.

As used herein, a "portion thereof" refers to a particular region of a protein, such as an extracellular portion, a transmembrane portion or an intracellular portion, or refers to a domain, motif, or fragment of a protein or protein region that retains the function associated with the domain, motif, or fragment of the protein or the protein region. For example, a portion thereof of a cytokine binding domain means a fragment of this domain that is still capable of binding the cytokine.

In certain embodiments, a fusion protein may contain a "linker," which can provide a spacer function to facilitate the interaction of two single chain fusion proteins, or positioning of one or more binding domains, so that the resulting polypeptide structure maintains a specific binding affinity to a target molecule or maintains signaling activity (*e.g*., effector domain activity) or both. Exemplary linkers include from one to about ten repeats of GlyₓSer_{y}, wherein x and y are independently an integer from 1 to 5.

"Junction amino acids" or "junction amino acid residues" refer to one or more (*e.g*., about 2-20) amino acid residues between two adjacent motifs, regions, or domains of a fusion protein, such as between a binding domain and an adjacent hydrophobic component, or on one or both ends of a hydrophobic component. Junction amino acids may result from the construct design of a fusion protein (e.g., amino acid residues resulting from the use of a restriction enzyme site during the construction of a nucleic acid molecule encoding a fusion protein). In certain embodiments, junction amino acids form a linker, such as those having from one to about ten repeats of GlyₓSer_{y}, wherein x and y are independently an integer from 1 to 5.

As used herein, an "immune system cell" means any cell of the immune system that originates from a hematopoietic stem cell in the bone marrow, which gives rise to two major lineages, a myeloid progenitor cell (which give rise to myeloid cells such as monocytes, macrophages, dendritic cells, megakaryocytes and granulocytes) and a lymphoid progenitor cell (which give rise to lymphoid cells such as T cells, B cells and natural killer (NK) cells). Exemplary immune system cells include a CD4+ T cell, a CD8+ T cell, a CD4- CD8- double negative T cell, a γδ T cell, a regulatory T cell, a natural killer cell, and a dendritic cell. Macrophages and dendritic cells may be referred to as "antigen presenting cells" or "APCs," which are specialized cells that can activate T cells when a major histocompatibility complex (MHC) receptor on the surface of the APC complexed with a peptide interacts with a TCR on the surface of a T cell.

A "T cell" is an immune system cell that matures in the thymus and produces T cell receptors (TCRs). T cells can be naive (not exposed to antigen; increased expression of CD62L, CCR7, CD28, CD3, CD127, and CD45RA, and decreased expression of CD45RO as compared to T_{CM}), memory T cells (T_{M}) (antigen-experienced and long-lived), and effector cells (antigen-experienced, cytotoxic). T_{M} can be further divided into subsets of central memory T cells (T_{CM}, increased expression of CD62L, CCR7, CD28, CD127, CD45RO, and CD95, and decreased expression of CD54RA as compared to naive T cells) and effector memory T cells (T_{EM}, decreased expression of CD62L, CCR7, CD28, CD45RA, and increased expression of CD127 as compared to naive T cells or T_{CM}). Effector T cells (T_{E}) refers to antigen-experienced CD8+ cytotoxic T lymphocytes that have decreased expression of CD62L ,CCR7, CD28, and are positive for granzyme and perforin as compared to T_{CM}. Other exemplary T cells include regulatory T cells, such as CD4+ CD25+ (Foxp3+) regulatory T cells and Treg17 cells, as well as Tr1, Th3, CD8+CD28-, and Qa-1 restricted T cells.

"T cell receptor" (TCR) refers to a molecule found on the surface of T cells (or T lymphocytes) that, in association with CD3, is generally responsible for recognizing antigens bound to major histocompatibility complex (MHC) molecules. The TCR has a disulfide-linked heterodimer of the highly variable α and β chains (also known as TCRα and TCRβ, respectively) in most T cells. In a small subset of T cells, the TCR is made up of a heterodimer of variable γ and δ chains (also known as TCRγ and TCRδ, respectively). Each chain of the TCR is a member of the immunoglobulin superfamily and possesses one N-terminal immunoglobulin variable domain, one immunoglobulin constant domain, a transmembrane region, and a short cytoplasmic tail at the C-terminal end (*see* Janeway et al., Immunobiology: The Immune System in Health and Disease, 3rd Ed., Current Biology Publications, p. 4:33, 1997). TCR, as used in the present disclosure, may be from various animal species, including human, mouse, rat, cat, dog, goat, horse, or other mammals. TCRs may be cell-bound (*i.e.,* have a transmembrane region or domain) or in soluble form.

"Major histocompatibility complex molecules" (MHC molecules), which is used interchangeably and is understood to also refer to the human counterpart human leukocyte antigen (HLA molecules), refer to glycoproteins that deliver peptide antigens to a cell surface. MHC class I molecules are heterodimers consisting of a membrane spanning α chain (with three α domains) and a non-covalently associated β2 microglobulin. MHC class II molecules are composed of two transmembrane glycoproteins, α and β, both of which span the membrane. Each chain has two domains. MHC (HLA) class I molecules deliver peptides originating in the cytosol to the cell surface, where peptide:MHC (or peptide:HLA in humans) complex is recognized by CD8⁺ T cells. MHC (HLA) class II molecules deliver peptides originating in the vesicular system to the cell surface, where they are recognized by CD4⁺ T cells. An MHC molecule may be from various animal species, including human, mouse, rat, or other mammals.

"Nucleic acid molecule", or polynucleotide, may be in the form of RNA or DNA, which includes cDNA, genomic DNA, and synthetic DNA. A nucleic acid molecule may be double stranded or single stranded, and if single stranded, may be the coding strand or non-coding (anti-sense strand). A coding molecule may have a coding sequence identical to a coding sequence known in the art or may have a different coding sequence, which, as the result of the redundancy or degeneracy of the genetic code, or by splicing, can encode the same polypeptide.

Variants of the nucleic acid molecules or polynucleotides of this disclosure are also contemplated. Variant polynucleotides are at least 90%, and preferably 95%, 99%, or 99.9% identical to one of the polynucleotides of defined sequence as described herein, or that hybridizes to one of those polynucleotides of defined sequence under stringent hybridization conditions of 0.015M sodium chloride, 0.0015M sodium citrate at about 65-68°C or 0.015M sodium chloride, 0.0015M sodium citrate, and 50% formamide at about 42°C. The polynucleotide variants retain the capacity to encode a binding domain or fusion protein thereof having the functionality described herein.

The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015M sodium chloride, 0.0015M sodium citrate at about 65-68°C or 0.015M sodium chloride, 0.0015M sodium citrate, and 50% formamide at about 42°C (*see* Sambrook et al., Molecular Cloning: A Laboratory Manual, 2nd Ed., Cold Spring Harbor Laboratory, Cold Spring Harbor, N.Y., 1989).

More stringent conditions (such as higher temperature, lower ionic strength, higher formamide, or other denaturing agent) may also be used; however, the rate of hybridization will be affected. In instances wherein hybridization of deoxyoligonucleotides is concerned, additional exemplary stringent hybridization conditions include washing in 6x SSC, 0.05% sodium pyrophosphate at 37°C (for 14-base oligonucleotides), 48°C (for 17-base oligonucleotides), 55°C (for 20-base oligonucleotides), and 60°C (for 23-base oligonucleotides).

A "vector" is a nucleic acid molecule that is capable of transporting another nucleic acid. Vectors may be, for example, plasmids, cosmids, viruses, or phage. An "expression vector" is a vector that is capable of directing the expression of a protein encoded by one or more genes carried by the vector when it is present in the appropriate environment.

"Retroviruses" are viruses having an RNA genome. "Gammaretrovirus" refers to a genus of the retroviridae family. Exemplary gammaretroviruses include mouse stem cell virus, murine leukemia virus, feline leukemia virus, feline sarcoma virus, and avian reticuloendotheliosis viruses.

"Lentivirus" refers to a genus of retroviruses that are capable of infecting dividing and non-dividing cells. Several examples of lentiviruses include HIV (human immunodeficiency virus: including HIV type 1, and HIV type 2); equine infectious anemia virus; feline immunodeficiency virus (FIV); bovine immune deficiency virus (BIV); and simian immunodeficiency virus (SIV).

The terms "identical" or "percent identity," in the context of two or more polypeptide or nucleic acid molecule sequences, means two or more sequences or subsequences that are the same or have a specified percentage of amino acid residues or nucleotides that are the same over a specified region (e.g., 60%, 65%, 70%, 75%, 80%, 85%, 90%, 91%, 92%, 93%, 94%, 95%, 96%, 97%, 98%, 99%, or 100% identity), when compared and aligned for maximum correspondence over a comparison window, or designated region, as measured using methods known in the art, such as a sequence comparison algorithm, by manual alignment, or by visual inspection. For example, preferred algorithms suitable for determining percent sequence identity and sequence similarity are the BLAST and BLAST 2.0 algorithms, which are described in Altschul et al. (Nucleic Acids Res. 25:3389, 1977) and Altschul et al. (J. Mol. Biol. 215:403, 1990), respectively.

"Treat" or "treatment" or "ameliorate" refers to medical management of a disease, disorder, or condition of a subject (*e.g*., a human or non-human mammal, such as a primate, horse, dog, mouse, or rat). In general, an appropriate dose or treatment regimen comprising a host cell expressing a fusion protein of this disclosure, and optionally an adjuvant or adjunctive therapy, is administered in an amount sufficient to elicit a therapeutic or prophylactic benefit. Therapeutic or prophylactic/preventive benefit includes improved clinical outcome; lessening or alleviation of symptoms associated with a disease; decreased occurrence of symptoms; improved quality of life; longer disease-free status; diminishment of extent of disease, stabilization of disease state; delay of disease progression; remission; survival; prolonged survival; or any combination thereof.

A "therapeutically effective amount" or "effective amount" of a fusion protein or cell expressing a fusion protein of this disclosure, in the context of a disease or condition being treated, refers to that amount of fusion protein or number of cells sufficient to result in amelioration of one or more symptoms of the disease being treated in a statistically significant manner (*e.g*., reducing infection, reducing tumor size, inhibiting cancer growth or the like).

### Nucleic Acids, Fusion Proteins, and Host Cells

Nucleic acid molecules that encode any one or more of the fusion proteins described herein, wherein the cytokine binding domain may not be an IL-2 binding domain, can be inserted into an appropriate vector (*e.g*., viral vector or non-viral plasmid vector) for introduction in a host cell of interest (*e.g*., T cell).

In some embodiments, the encoded intracellular component is comprised of an intracellular portion, intracellular signaling domain or portion thereof from an IL-2Rγ, IL-2Rβ, IL-4R, IL-7R, IL-9R, IL-15R, IL-21R, or any combination thereof. In related embodiments, the encoded intracellular component is comprised of an intracellular portion, intracellular signaling domain or portion thereof from a human IL-2Rγ, human IL-2Rβ, human IL-4R, human IL-7R, human IL-9R, human IL-15R, human IL-21R, or any combination thereof.

In certain embodiments, a polynucleotide encodes (a) a first fusion protein and all or portion of the cytokine binding domain is a first portion of the cytokine binding domain, and (b) a second fusion protein comprising a second portion of the cytokine binding domain, a second transmembrane domain and a second intracellular component comprising a second IL-2R chain signaling domain or signaling portion thereof, and optionally further comprising a polynucleotide encoding an antigen receptor or portion thereof or an antigen binding protein, such as an antigen-specific TCR or CAR.

In further embodiments, the polynucleotide encoding a fusion protein comprises a transmembrane domain disposed between an extracellular component comprising a cytokine binding domain or portion thereof, and an intracellular component comprising an IL-2Rγ intracellular portion, intracellular signaling domain or a portion thereof, optionally a human IL-2Rγ. In particular embodiments, a polynucleotide encodes a fusion protein comprising a transmembrane domain disposed between an extracellular component comprising a cytokine binding domain or portion thereof, and an intracellular component, wherein a non-cytokine binding portion of the extracellular component, the transmembrane domain, and the intracellular component of the encoded fusion protein has at least 85%, at least 90%, at least 95%, at least 96%, at least 97%, at least 98%, or at least 99% amino acid sequence identity to the amino acid sequence set forth in SEQ ID NO.: 10. In still further embodiments, the encoded non-cytokine binding extracellular portion, transmembrane domain, and intracellular portion of the encoded fusion protein is comprised of or consists of the amino acid sequence set forth in SEQ ID NO.: 10.

In certain embodiments, any of the aforementioned polynucleotides encode a fusion protein comprising an extracellular component comprising a cytokine binding domain and a portion of an IL-2R chain comprising a non-cytokine binding extracellular portion, a transmembrane domain of an IL-2R chain, and an intracellular portion of an IL-2R chain, such as an IL-2Rγ or human IL-2Rγ. In further embodiments, the non-cytokine binding extracellular portion, transmembrane domain, and intracellular portion of the IL-2R chain is encoded by a polynucleotide having at least 80%, at least 85%, at least 90%, at least 95%, or at least 98% identity to nucleotides 961-1,308 of SEQ ID NO.:6. In still further embodiments, the non-cytokine binding extracellular portion, transmembrane domain, and intracellular portion of the IL-2R chain is encoded by a polynucleotide comprising or consisting of nucleotides 961-1,308 of SEQ ID NO.:6.

In yet further embodiments, the polynucleotide comprises encoding a fusion protein comprising a transmembrane domain disposed between an extracellular component comprising a cytokine binding domain or portion thereof, and an intracellular component comprising an IL-2Rβ intracellular portion, intracellular signaling domain or a portion thereof, optionally a human IL-2Rβ. In particular embodiments, a polynucleotide encodes a fusion protein comprising a transmembrane domain disposed between an extracellular component comprising a cytokine binding domain or portion thereof, and an intracellular component, wherein the transmembrane domain and the intracellular component of the encoded fusion protein has at least 85%, at least 90%, at least 95%, at least 96%, at least 97%, at least 98%, or at least 99% amino acid sequence identity to the sequence set forth in SEQ ID NO.:12. In still further embodiments, the encoded transmembrane domain and intracellular portion of the encoded fusion protein is comprised of or consists of the amino acid sequence set forth in SEQ ID NO.:12.

In certain embodiments, any of the aforementioned polynucleotides encode a fusion protein comprising an extracellular component comprising a cytokine binding domain, a transmembrane domain of an IL-2R chain, and an intracellular portion of an IL-2R chain, such as an IL-2Rβ or human IL-2Rβ. In further embodiments, the transmembrane domain and intracellular portion of the IL-2R chain is encoded by a polynucleotide having at least 80%, at least 85%, at least 90%, at least 95%, or at least 98% identity to nucleotides 1,315-2,250 of SEQ ID NO.:7. In still further embodiments, the non-cytokine binding extracellular portion, transmembrane domain, and intracellular portion of the IL-2R chain is encoded by a polynucleotide comprising or consisting of nucleotides 1,315-2,250 of SEQ ID NO.:7.

In any of the aforementioned embodiments, the encoded extracellular component comprises an extracellular domain or portion thereof of a CSF2RA (also referred to as GM-CSFR or CSF2R), CSF2RB (also referred to as IL3RB, IL5RB, CD131), CSF1R (also referred to as M-CSFR or CSFR), CSF3R (also referred to as G-CSFR or CD114), CXCR2 (also referred to as IL8RA, IL8RB, IL8R2 or CD182), or CCR8 (also referred to as CY6 or TER1). In further embodiments, the encoded extracellular component comprises an extracellular domain or portion thereof of a human CSF2RA, human CSF2RB, human CSF1R, human CSF3R, human CXCR2, or human CCR8.

In any of the aforementioned embodiments, the encoded cytokine binding domain or binding portion thereof specifically binds to a GM-CSF (also referred to as CSF2), M-CSF (also referred to as CSF1), G-CSF (also referred to as CSF3), CXCL1, CXCL2, or CCL1. In further embodiments, the encoded cytokine binding domain or binding portion thereof specifically binds to a human GM-CSF, a human M-CSF, a human G-CSF, a human CXCL1, a human CXCL2, or a human CCL1.

In certain embodiments, any of the aforementioned polynucleotides encode a fusion protein comprising a cytokine binding domain specific for CSF2, such as an extracellular portion of a CSF2RA or CSF2RB. In further embodiments, the extracellular portion of a CSF2RA is encoded by a polynucleotide having at least 80%, at least 85%, at least 90%, at least 95%, or at least 98% identity to nucleotides 1-960 of SEQ ID NO.:6. In still further embodiments, the extracellular portion of a CSF2RA is encoded by a polynucleotide comprising or consisting of nucleotides 1-960 of SEQ ID NO.:6. In related embodiments, the encoded extracellular portion of a CSF2RA is at least 85%, at least 90%, at least 95%, at least 96%, at least 97%, at least 98%, or at least 99% identical to the amino acid sequence set forth in SEQ ID NO.:9. In still further embodiments, the encoded extracellular portion of a CSF2RA is comprised of or consists of the amino acid sequence set forth in SEQ ID NO.:9. In other embodiments, the extracellular portion of a CSF2RB is encoded by a polynucleotide having at least 80%, at least 85%, at least 90%, at least 95%, or at least 98% identity to nucleotides 1-1,314 of SEQ ID NO.:7. In still further embodiments, the extracellular portion of a CSF2RB is encoded by a polynucleotide comprising or consisting of nucleotides 1-1,314 of SEQ ID NO.:6. In related embodiments, the encoded extracellular portion of a CSF2RB is at least 85%, at least 90%, at least 95%, at least 96%, at least 97%, at least 98%, or at least 99% identical to the amino acid sequence set forth in SEQ ID NO.:11. In yet other embodiments, the encoded extracellular portion of a CSF2RB is comprised of or consists of the amino acid sequence set forth in SEQ ID NO.:11.

In certain embodiments, any of the aforementioned polynucleotides encode a fusion protein comprising a transmembrane domain, such as a transmembrane domain of an IL-2RG, IL-2RB, CSF2RA, CSF2RB, CSF1R, CSF3R, CXCR2, CCR8, IL-2RA, IL-4R, IL-7R, IL-9R, IL-15R, IL-21R, CD2, CD3ε, CD3δ, CD3ζ, CD25, CD27, CD28, CD40, CD79A, CD79B, CD80, CD86, CD95 (Fas), CD134 (OX40), CD137 (4-1BB), CD150 (SLAMF1), CD152 (CTLA4), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR2, Ryk, Slp76, SIRPα, pTα, TCRα, TCRβ, TIM3, TRIM, LPA5, or Zap70. In some embodiments, the encoded transmembrane domain is of ahuman IL-2RG, a human IL-2RB, a human CSF2RA, a human CSF2RB, a human CSF1R, a human CSF3R, a human CXCR2, a human CCR8, a human IL-2RA, a human IL-4R, a human IL-7R, a human IL-9R, a human IL-15R, a human IL-21R, a human CD2, a human CD3ε, a human CD3δ, a human CD3ζ, a human CD25, a human CD27, a human CD28, a human CD40, a human CD79A, a human CD79B, a human CD80, a human CD86, a human CD95 (Fas), a human CD134 (OX40), a human CD137 (4-1BB), a human CD150 (SLAMF1), a human CD152 (CTLA4), a human CD200R, a human CD223 (LAG3), a human CD270 (HVEM), a human CD272 (BTLA), a human CD273 (PD-L2), a human CD274 (PD-L1), a human CD278 (ICOS), a human CD279 (PD-1), a human CD300, a human CD357 (GITR), a human A2aR, a human DAP10, a human FcRα, a human FcRβ, a human FcRγ, a human Fyn, a human GAL9, a human KIR, a human Lck, a human LAT, a human LRP, a human NKG2D, a human NOTCH1, a human NOTCH2, a human NOTCH3, a human NOTCH4, a human PTCH2, a human ROR1, a human ROR2, a human Ryk, a human Slp76, a human SIRPα, a human pTα, a human TCRα, a human TCRβ, a human TIM3, a human TRIM, a human LPA5, or a human Zap70. In particular embodiments, any of the aforementioned polynucleotides encode a fusion protein comprising a transmembrane domain that is at least 90%, at least 95%, at least 96%, at least 97%, at least 98%, or at least 99% identical to the amino acid sequence set forth in SEQ ID NO.:22 or 23. In other embodiments, the encoded transmembrane domain comprises or consists of the amino acid sequence set forth in SEQ ID NO.:22 or 23.

In the case of a polynucleotide encoding an antigen binding protein, such an encoded antigen binding protein may comprise a transmembrane domain according to any of the aforementioned transmembrane domain embodiments.

In certain embodiments, the polynucleotide encoding a fusion protein comprises a transmembrane domain disposed between an extracellular component comprising a cytokine binding domain or portion thereof, and an intracellular component; wherein the encoded extracellular component comprises an extracellular portion of a CSF2RA and a non-cytokine binding extracellular portion of IL-2Rγ, and the encoded transmembrane domain and encoded intracellular component comprise a portion of IL-2Rγ. In further embodiments, a polynucleotide encodes a fusion protein having at least 90%, at least 95%, at least 96%, at least 97%, at least 98%, or at least 99% identity with the amino acid sequence as set forth in SEQ ID NO.:1. In still further embodiments, a polynucleotide encodes a fusion protein comprising or consisting of the amino acid sequence as set forth in SEQ ID NO.:1.

In certain embodiments, the polynucleotide encoding a fusion protein comprises a transmembrane domain disposed between an extracellular component comprising a cytokine binding domain or portion thereof, and an intracellular component; wherein the encoded extracellular component comprises an extracellular portion of a CSF2RB, and the encoded transmembrane domain and encoded intracellular component comprise a portion of IL-2Rβ. In further embodiments, a polynucleotide encodes a fusion protein having at least 90%, at least 95%, at least 96%, at least 97%, at least 98%, or at least 99% identity with the amino acid sequence as set forth in SEQ ID NO.:2. In further embodiments, a polynucleotide encodes a fusion protein comprising or consisting of the amino acid sequence as set forth in SEQ ID NO.:2.

The first 22 amino acids of SEQ ID NOS.:1 and 9, and the first 16 amino acids of SEQ ID NOS.:2 and 11, correspond to a signal sequence for human CSF2RA and CSF2RB, respectively. In certain embodiments wherein a fusion protein of SEQ ID NOS.:1, 2, 9 and 11 is expressed on the surface of a host cell will be a mature protein - that is, the mature protein lacks the signal sequence. For example, a mature protein of CSF2RA according to SEQ ID NO.: 9 corresponds to amino acids 23-320 of SEQ ID NO. :9, and a mature protein of CSF2RB according to SEQ ID NO.:11 corresponds to amino acids 17-438 of SEQ ID NO.:11.

In some embodiments, a polynucleotide encodes a fusion protein comprising an intracellular signaling domain or functional fragment or portion thereof from IL-2R, such as IL-2Rγ or IL-2Rβ. In other embodiments, a polynucleotide encodes an antigen binding protein coprising an intracellular signaling domain or functional fragment or portion thereof from a CD3ε, CD3δ, CD3ζ, CD25, CD27, CD28, CD40, CD47, CD79A, CD79B, CD134 (OX40), CD137 (4 1BB), CD150 (SLAMF1), CD278 (ICOS), CD357 (GITR), CARD11, DAP10, DAP12, FcRα, FcRβ, FcRγ, Fyn, Lck, LAT, LRP, NKG2D, NOTCH1, NOTCH2, NOTCH3, NOTCH4, ROR2, Ryk, Slp76, pTα, TCRα, TCRβ, TRIM, Zap70, PTCH2, or any combination thereof. In some embodiments, an encoded intracellular signaling domain or functional fragment or portion thereof of a fusion protein or antigen binding protein does not comprise a CD3ζ.

As used herein, the term "recombinant" or "non-natural" refers to an organism, microorganism, cell, nucleic acid molecule, or vector that includes at least one genetic alteration or has been modified by introduction of an exogenous nucleic acid molecule, wherein such alterations or modifications are introduced by genetic engineering. Genetic alterations include, for example, modifications introducing expressible nucleic acid molecules encoding proteins, fusion proteins or enzymes, or other nucleic acid molecule additions, deletions, substitutions or other functional disruption of a cell's genetic material. Additional modifications include, for example, non-coding regulatory regions in which the modifications alter expression of a gene or operon. In certain embodiments, a cell, such as a T cell, obtained from a subject may be converted into a non-natural or recombinant cell (*e.g.,* a non-natural or recombinant T cell) by introducing a nucleic acid that encodes a fusion protein as described herein and whereby the cell expresses a fusion protein.

In certain embodiments, a polynucleotide encodes a plurality of fusion proteins, a plurality of antigen binding proteins, or a combination thereof, wherein two or more of the plurality of fusion proteins, antigen binding proteins, or combinations thereof are separated by a cleavage site. In some embodiments, a cleavage site comprises a protease cleavage site of 2 to about 20 amino acids amino-terminal to a fusion protein or antigen binding protein, a protease cleavage site of 2 to about 20 amino acids carboxy-terminal to a fusion protein or antigen binding protein, a self-cleaving amino acid sequence, or a combination thereof.

In certain embodiments, an encoded cleavage site is a self-cleaving amino acid sequence comprising a 2A peptide from porcine teschovirus-1 (P2A) (SEQ ID NO.:13), *Thosea asigna* virus (T2A) (SEQ ID NO.:14), equine rhinitis A virus (E2A) (SEQ ID NO.:15), foot-and-mouth disease virus (F2A) (SEQ ID NO.:16), or any combination thereof (*see, e.g.,* Kim et al., PLOS One 6:e18556, 2011). In further embodiments, a polynucleotide that encodes a plurality of fusion proteins, a plurality of antigen binding proteins, or a combination thereof includes a sequence encoding a self-cleaving peptide located between two or more of the proteins, which may be: (a) a P2A peptide encoded by a polynucleotide as set forth in SEQ ID NO.:17; (b) a P2A peptide encoded by a codon optimized polynucleotide as set forth in SEQ ID NO.:18; (c) a T2A peptide is encoded by a polynucleotide as set forth in SEQ ID NO.:19; (d) an E2A peptide is encoded by a polynucleotide as set forth in SEQ ID NO.:20; (e) a F2A peptide is encoded by a polynucleotide as set forth in SEQ ID NO.:21; or (f) any combination thereof.

In certain embodiments, a polynucleotide of this disclosure a first fusion protein, a second fusion protein and optionally an antigen binding protein, wherein the first fusion protein comprises a transmembrane domain disposed between an extracellular component comprising a cytokine binding domain or portion thereof, and an intracellular component that comprises,consists of, or has at least 90% identity to, an IL-2Rγ, optionally a human IL-2Rγ, intracellular portion or intracellular signaling domain or portion thereof, or optionally has at least 85%, at least 90%, at least 95%, at least 96%, at least 97%, at least 98%, or at least 99% amino acid sequence identity to the amino acid sequence set forth in SEQ ID NO.:10. In some embodiments, a polynucleotide encodes a first fusion protein having at least 90%, at least 95%, at least 96%, at least 97%, at least 98%, or at least 99% identity with the amino acid sequence as set forth in SEQ ID NO.:1. In still further embodiments, a polynucleotide encodes a first fusion protein comprising or consisting of the amino acid sequence as set forth in SEQ ID NO.:1.

In further embodiments, a polynucleotide of this disclosure encodes a first fusion protein, a second fusion protein and optionally an antigen binding protein, wherein the encoded first fusion protein comprises a transmembrane domain disposed between an extracellular component comprising a cytokine binding domain or portion thereof, and an intracellular component that comprises,consists of, or has at least 90% identity to, an IL-2Rβ, optionally a human IL-2Rβ, intracellular portion or intracellular signaling domain or portion thereof, or optionally has at least 85%, at least 90%, at least 95%, at least 96%, at least 97%, at least 98%, or at least 99% amino acid sequence identity to the amino acid sequence set forth in SEQ ID NO.: 12. In some embodiments, a polynucleotide encodes a first fusion protein having at least 90%, at least 95%, at least 96%, at least 97%, at least 98%, or at least 99% identity with the amino acid sequence as set forth in SEQ ID NO.:2. In further embodiments, a polynucleotide encodes a first fusion protein comprising or consisting of the amino acid sequence as set forth in SEQ ID NO.:2.

In still further embodiments, a polynucleotide of this disclosure encodes a first fusion protein, a second fusion protein, and optionally an antigen binding protein, wherein the encoded first fusion protein has at least 85%, at least 90%, at least 95%, at least 96%, at least 97%, at least 98%, or at least 99% amino acid sequence identity to the amino acid sequence set forth in SEQ ID NO.:1 or set forth in amino acids 23-435 (mature fusion protein) of SEQ ID NO.: 1; the encoded second fusion protein has at least 90%, at least 95%, at least 96%, at least 97%, at least 98%, or at least 99% amino acid sequence identity to the amino acid sequence set forth in SEQ ID NO.:2 or set forth in amino acids 17-749 (mature fusion protein) of SEQ ID NO.:2; and the optional antigen binding protein comprises an antigen-specific TCR or an antigen-specific CAR, wherein the antigen is optionally a cancer-specific antigen, such as a WT-1, mesothelin, ROR1 or cyclin-A1 antigen.

In each of these embodiments, a first fusion protein, a second fusion protein, and an antigen binding protein may be all encoded by a single polynucleotide, or they all may be encoded by two polynucleotides (*e.g*., a first polynucleotide encodes the first fusion protein and a second polynucleotide encodes the second fusion protein and the antigen binding protein; or a first polynucleotide encodes the second fusion protein and a second polynucleotide encodes the first fusion protein and the antigen binding protein; or a first polynucleotide encodes the first fusion protein and second fusion protein, and a second polynucleotide encodes the antigen binding protein, or any combination thereof).

In certain embodiments, a single polynucleotide encodes a first fusion protein of this disclosure and a second fusion protein of this disclosure, wherein a polynucleotide encoding a a self-cleaving peptide as set forth in any one of SEQ ID NOS.: 17-21 is disposed between and links the polynucleotide encoding the first fusion protein with the polynucleotide encoding the second fusion protein. In particular embodiments, the first fusion protein is encoded by a polynucleotide having at least 80%, at least 85%, at least 90%, at least 95%, or at least 98% identity to the nucleotide sequence set forth in SEQ ID NO.:6, and the second fusion protein is encoded by a polynucleotide having at least 80%, at least 85%, at least 90%, at least 95%, or at least 98% identity to the nucleotide sequence set forth in SEQ ID NO.:7. In further embodiments, the first fusion protein is encoded by a polynucleotide comprising or consisting of the nucleotide sequence set forth in SEQ ID NO.:6, and the second fusion protein is encoded by a polynucleotide comprising or consisting of the nucleotide sequence set forth in SEQ ID NO.:7.

In any of the aforementioned embodiments, a polynucleotide encoding a fusion protein, an antigen binding protein or both, of this disclosure, may be codon optimized to enhance or maximize expression in certain types of cells, such as T cells (Scholten et al., Clin. Immunol. 119: 135-145, 2006).

Any of the polynucleotides of this disclosure may be contained in a vector or delivered to a host cell (*e.g*., T cell) via a vector. A vector that encodes a core virus is referred to herein as a "viral vector." There are a large number of available viral vectors suitable for use with the compositions of the instant disclosure, including those identified for human gene therapy applications (*see* Pfeifer and Verma, Ann. Rev. Genomics Hum. Genet. 2:177, 2001). Suitable viral vectors include vectors based on RNA viruses, such as retrovirus-derived vectors, *e.g*., Moloney murine leukemia virus (MLV)-derived vectors, and include more complex retrovirus-derived vectors, *e.g*., lentivirus-derived vectors. HIV-1-derived vectors belong to this category. Other examples include lentivirus vectors derived from HIV-2, FIV, equine infectious anemia virus, SIV, and Maedi-Visna virus (ovine lentivirus). Methods of using retroviral and lentiviral viral vectors and packaging cells for transducing mammalian host cells with viral particles containing chimeric antigen receptor transgenes are known in the art and have been previous described, for example, in U.S. Patent 8,119,772; Walchli et al., PLoS One 6:327930, 2011; Zhao et al., J. Immunol. 174:4415, 2005; Engels et al., Hum. Gene Ther. 14:1155, 2003; Frecha et al., Mol. Ther. 18:1748, 2010; Verhoeyen et al., Methods Mol. Biol. 506:97, 2009. Retroviral and lentiviral vector constructs and expression systems are also commercially available.

In certain embodiments, a viral vector is used to introduce a non-endogenous polynucleotide encoding a fusion protein as disclosed herein or a non-endogenous polynucleotide encoding an antigen binding protein specific for a target as disclosed herein, or both. A viral vector may be a retroviral vector or a lentiviral vector. A viral vector may also include nucleic acid sequences encoding a marker for transduction. Transduction markers for viral vectors are known in the art and include selection markers, which may confer drug resistance, or detectable markers, such as fluorescent markers or cell surface proteins that can be detected by methods such as flow cytometry. In particular embodiments, a viral vector further comprises a gene marker for transduction comprising green fluorescent protein (GFP), an extracellular domain of human CD2, or a truncated human EGFR (huEGFRt; *see* Wang et al., Blood 118:1255, 2011). When a viral vector genome comprises a plurality of nucleic acid sequences to be expressed in a host cell as separate transcripts, the viral vector may also comprise additional sequences between the two (or more) transcripts allowing bicistronic or multicistronic expression. Examples of such sequences used in viral vectors include internal ribosome entry sites (IRES), furin cleavage sites, viral 2A peptide, or any combination thereof.

Other vectors also can be used for polynucleotide delivery including DNA viral vectors, including, for example adenovirus-based vectors and adeno-associated virus (AAV)-based vectors; vectors derived from herpes simplex viruses (HSVs), including amplicon vectors, replication-defective HSV and attenuated HSV (Krisky et al., Gene Ther. 5: 1517, 1998).

Other vectors recently developed for gene therapy uses can also be used with the compositions and methods of this disclosure. Such vectors include those derived from baculoviruses and α-viruses (Jolly, D J. 1999. Emerging Viral Vectors. pp 209-40 in Friedmann T. ed. The Development of Human Gene Therapy. New York: Cold Spring Harbor Lab), or plasmid vectors (such as sleeping beauty or other transposon vectors). In some embodiments, a viral or plasmid vector further comprises a gene marker for transduction (*e.g*. green fluorescent protein, huEGFRt).

In some embodiments, a vector comprises a polynucleotide as disclosed herein that encodes more than one fusion protein, and containing a polynucleotide that encodes an antigen-binding protein of this disclosure. For example, a vector may contain a polynucleotide that encodes two different fusion proteins, and containing a polynucleotide that encodes an antigen-binding protein of this disclosure.

In some embodiments, the antigen-specific TCR is specific to a HLA (MHC) class I restricted antigen. Embodiments wherein the cancer-specific antigen comprises WT-1, mesothelin, ROR1 or cyclin-A1 are also within the scope of this disclosure.

Any of the polynucleotides disclosed herein are contained in a host cell, wherein the host cell expresses and produces a fusion protein and an antigen binding protein. In some embodiments, a host cell of this disclosure comprises a plurality of antigen binding proteins, such as both a TCR and a CAR.

In further aspects, the present disclosure provides a host cell comprising a first fusion protein, a second fusion protein and an antigen binding protein, wherein the first fusion protein comprises a transmembrane domain disposed between an extracellular component comprising a cytokine binding domain or portion thereof, and an intracellular component that is comprised of, or has at least 90% identity to, an IL-2Rγ, optionally human IL-2Rγ, intracellular portion or intracellular signaling domain or portion thereof, or optionally has at least 85%, at least 90%, at least 95%, at least 96%, at least 97%, at least 98%, or at least 99% amino acid sequence identity to the amino acid sequence set forth in SEQ ID NO.: 10; wherein the second fusion protein comprises a transmembrane domain disposed between an extracellular component comprising a cytokine binding domain, or portion thereof, and an intracellular component is comprised of, or has at least 90% identity to, an IL-2R β, optionally human IL-2Rβ, intracellular signaling domain or portion thereof and/or a signaling domain or portion thereof of an IL-4R, IL-7R, IL-9R, IL-15R or IL-21R chain, or optionally has at least 85%, at least 90%, at least 95%, at least 96%, at least 97%, at least 98%, or at least 99% amino acid sequence identity to the amino acid sequence set forth in SEQ ID NO.: 12; and wherein the antigen binding protein comprises a T cell receptor (TCR); a chimeric antigen receptor (CAR); and optionally comprises a plurality of antigen binding proteins. In some embodiments, a host cell of this disclosure comprises a plurality of antigen binding proteins, such as both a TCR and a CAR.

In further aspects, the present disclosure provides a host cell comprising a polynucleotide that encodes a first fusion protein, a second fusion protein, and an antigen binding protein, wherein the encoded first fusion protein has at least 85%, at least 90%, at least 95%, at least 96%, at least 97%, at least 98%, or at least 99% amino acid sequence identity to the amino acid sequence set forth in amino acids 23-435 (mature fusion protein) of SEQ ID NO.: 1; the encoded second fusion protein has at least 90%, at least 95%, at least 96%, at least 97%, at least 98%, or at least 99% amino acid sequence identity to the amino acid sequence set forth in amino acids 17-749 (mature fusion protein) of SEQ ID NO.:2; and the optional antigen binding protein comprises an antigen-specific TCR or an antigen-specific CAR, wherein the antigen is a cancer-specific antigen, such as a WT-1, mesothelin, ROR1 or cyclin-A1 antigen.

In certain embodiments, a host cell containing a polynucleotide of this disclosure comprises at least two encoded fusion proteins that are capable of associating to form a heteromultimer on the host cell surface, optionally wheren the heteromultimer on the host cell surface is a heterodimer or heterotrimer.

In further embodiments, the fusion proteins each comprise a different extracellular component, wherein the different extracellular components are capable of associating with each other to form a functional cytokine binding domain. In particular embodiments, one of the different encoded extracellular components is comprised of, or has at least 90% amino acid identity to the amino acid sequence of, a CSF2Rα, optionally a human CSF2Rα, extracellular portion, extracellular cytokine binding domain or portion thereof, or optionally has at least 90% amino acid identity to the amino acid sequence of SEQ ID NO.:9, and the other different extracellular component is comprised of, or has at least 90% amino acid identity to the amino acid sequence of, a CSF2Rβ, optionally human CSF2Rβ, extracellular portion, extracellular cytokine binding domain or portion thereof, or optionally has at least 90% amino acid identity to the amino acid sequence of SEQ ID NO.:11. In any of these embodiments, the fusion proteins each comprise a different intracellular component, wherein the different intracellular components are capable of associating with each other to form a functional intracellular signaling domain. In further embodiments, at least one of the different intracellular components is comprised of, or has at least 90% identity to, an IL-2Rγ intracellular portion, intracellular signaling domain or portions thereof, or optionally has at least 90% amino acid sequence identity to the amino acid sequence of SEQ ID NO.:10. In yet further embodiments, at least one of the different intracellular components is comprised of, or has at least 90% identity to, an IL-2Rβ, IL-4RA, IL-7R, IL-15RA, or IL-21R intracellular portion, intracellular signaling domain or portions thereof, which in each case, individually, is optionally human-derived, or optionally has at least 90% identity to SEQ ID NO.:12. the TCR binds to an antigen::HLA complex with high affinity, such as at a Kₐ equal to or greater than 10⁷ M⁻¹. In further embodiments, the encoded antigen-specific TCR is heterologous to the host cell, or to a subject to whom the host cell will be administered. In particular embodiments, the encoded TCR is specific to a HLA class I restricted antigen. In some embodiments, the antigen binding protein is specific for WT-1, mesothelin, ROR1 or cyclin-A1. In some embodiments, the TCR is WT-1 specific TCR designated as C4.

In still further embodiments, a host cell containing a polynucleotide that encodes a fusion protein or a pluralityof fusion proteins of this disclosure, may further contain a polynucleotide that encodes an antigen binding protein, wherein the antigen binding protein is a CAR. Exemplary CARs expressed the host cell may comprise an extracellular antigen binding domain and an intracellular signaling domain capable of delivering a primary signal to a T cell and optionally a costimulatory domain; or the intracellular signaling domain comprises an intracellular signaling domain of a costimulatory molecule, such as from CD28, CD137 (4-1BB), or ICOS. In some embodiments, the encoded intracellular signaling domain comprises an intracellular signaling domain of a CD3ε, CD3δ, CD3ζ, CD25, CD27, CD28, CD40, CD47, CD79A, CD79B, CD134 (OX40), CD137 (4-1BB), CD150 (SLAMF1), CD278 (ICOS), CD357 (GITR), CARD11, DAP10, DAP12, FcRα, FcRβ, FcRγ, Fyn, Lck, LAT, LRP, NKG2D, NOTCH1, NOTCH2, NOTCH3, NOTCH4, ROR2, Ryk, Slp76, pTα, TCRα, TCRβ, TRIM, Zap70, PTCH2, or any combination thereof and/or wherein the intracellular signaling portion of the chimeric antigen receptor comprises a primary activation signaling domain, which optionally is derived from CD3 ζ, and does not comprise a costimulatory domain and/or does not comprise a CD28 signaling domain, a 4-1BB signaling domain and/or an ICOS signaling domain.

In certain embodiments, the encoded intracellular signaling domain comprises a costimulatory domain of: (a) a CD137 (4-1BB), CD27, CD28, ICOS, OX40 (CD134), or any combination thereof; (b) a CD137 (4-1BB) or CD28, or any combination thereof; (c) a CD28; or (d) a CD137 (4-1BB). In particular embodiments, the encoded intracellular signaling domain comprises a second intracellular signaling domain, such as an intracellular signaling domain of a CD137 (4-1BB).

In further embodiments, the encoded antigen binding domain of the CAR comprises an antibody binding fragment or scFv specific for the antigen. In some embodiments, a host cell as described herein comprises at least two antigen binding proteins, wherein the at least two antigen binding proteins include a TCR and a CAR. In certain embodiments, the expression of the fusion protein in a T cell comprising a TCR or chimeric antigen receptor specific for an antigen results in at least about a 1.5-fold, 2-fold, or 3-fold increase in survival, expansion, cytotoxicity, cytokine secretion, and/or response to multiple rounds of stimulation, by the T cell, in response to binding of the antigen and/or following administration to a subject, and/or results in at least about a 1.5-fold, 2-fold, or 3-fold increase in time of survival, disease-free survival, or amelioration of one or more disease symptom, of a subject to which the cell is administered, as compared to a cell substantially the same as the T cell but not containing the fusion protein.

Exemplary host cells for use with the fusion proteins, antigen binding proteins and polynucleotides encoding the same of this disclosure includes an immune system cell, such as a T cell. A T cell may be a CD4+ T cell or a CD8+ T cell.

In some embodiments, host cells capable of expressing a fusion protein of this disclosure on the cell surface are immune cells. In some embodiments, host cells capable of expressing a fusion protein of this disclosure on the cell surface are T cells, including primary cells or cell lines derived from human, mouse, rat, or other mammals. If obtained from a mammal, a T cell can be obtained from numerous sources, including blood, bone marrow, lymph node, thymus, or other tissues or fluids. A T cell may be enriched or purified. T cell lines are well known in the art, some of which are described in Sandberg et al., Leukemia 21:230, 2000. In certain embodiments, T cells that lack endogenous expression of TCRα and β chains are used. Such T cells may naturally lack endogenous expression of TCRα and β chains or may have been modified to block expression (e.g., T cells from a transgenic mouse that does not express TCR α and β chains or cells that have been manipulated to inhibit expression of TCR α and β chains) or to knockout TCRα chain, TCRβ chain, or both genes. In some embodiments, T cells may be engineered to express a TCR specific to a particular antigen.

In certain embodiments, a host cell transfected to express a fusion protein of this disclosure is a functional T cell, such as a virus-specific T cell, a tumor antigen specific cytotoxic T cell, a naive T cell, a memory stem T cell, a central or effector memory T cell, γδ T cells, or a CD4+ CD25+ regulatory T cell. In further embodiments, a nucleic acid molecule encoding a fusion protein of this disclosure is introduced into bulk CD8+ T cells, naive CD8+ T cells, CD8+ T_{CM} cells, CD8+ T_{EM} cells, or any combination thereof. In still further embodiments, a nucleic acid molecule encoding a fusion protein of this disclosure is introduced into bulk CD4+ T cells, naive CD4+ T cells, CD4+ T_{CM} cells, CD4+ T_{EM} cells, or any combination thereof. In other embodiments, a nucleic acid molecule encoding a fusion protein of this disclosure is introduced into a population of T cells enriched for naive CD8+ T cells and CD8+ T_{CM} cells. In still other embodiments, a nucleic acid molecule encoding a fusion protein of this disclosure is introduced into a population of T cells enriched for naïve CD4+ T cells and CD4+ T_{CM} cells. In any of the aforementioned embodiments, the T cells further contain a nucleic acid molecule encoding an engineered antigen-specific T cell receptor (TCR), an engineered antigen-specific high affinity TCR, an exogenous co-stimulatory molecule, a chimeric antigen receptor (CAR), or any combination thereof.

In certain embodiments, a host cell transfected to express a fusion protein of this disclosure is a functional natural killer cell.

One or more growth factor cytokines that promote proliferation of T cells expressing a fusion protein of this disclosure may be added to the culture used to expand T cells. The cytokines may be human or non-human. Exemplary growth factor cytokines that may be used promote T cell proliferation include GM-CSF, IL-2, IL-15, or the like.

In certain embodiments, a host T cell transfected to express a fusion protein of this disclosure is a CD4⁺ T cell that also expresses an antigen-specific high-affinity TCR specific to a HLA (MHC) class I restricted antigen (see Soto et al., Cancer Immunol Immunother. 62: 359-369, 2013).

In certain embodiments, a host T cell transfected to express a fusion protein of this disclosure also expresses a recombinant TCR specific to a cancer antigen. In some embodiments, the cancer antigen is a WT1. "WT1" refers to Wilm's tumor 1, a transcription factor that contains four zinc-finger motifs at the C-terminus and a proline/glutamine-rich DNA binding domain at the N-terminus. WT1 has an essential role in the normal development of the urogenital system and is mutated in a small subset of patients with Wilm's tumors. High expression of WT1 has been observed in various cancers, including, breast cancer, ovarian cancer, acute leukemias, vascular neoplasms, melanomas, colon cancer, lung cancer, thyroid cancer, bone and soft tissue sarcoma, and esophageal cancer. Alternative splicing has been noted for WT1.

In certain embodiments, a host T cell transfected to express a fusion protein of this disclosure also expresses a recombinant TCR specific to mesothelin. "Mesothelin" (MSLN) refers to a gene that encodes a precursor protein that is cleaved into two products, megakaryocyte potentiating factor and mesothelin. Megakaryocyte potentiation factor functions as a cytokine that can stimulate colony formation in bone marrow megakaryocytes. Mesothelin is a glycosylphosphatidylinositol-anchored cell-surface protein that may function as a cell adhesion protein. This protein is overexpressed in epithelial mesotheliomas, ovarian cancers and in specific squamous cell carcinomas. Alternative splicing results in multiple transcript variants.

In certain embodiments, a host T cell transfected to express a fusion protein of this disclosure also expresses a recombinant TCR specific to cyclin-A1.

In certain embodiments, a host T cell transfected to express a fusion protein of this disclosure also expresses a CAR.

### Uses

Diseases that may be treated with cells of the present invention include cancer, infectious diseases (viral, bacterial, protozoan infections), and immune diseases (e.g., autoimmune). Adoptive immune and gene therapy are promising treatments for various types of cancer (Morgan et al., Science 314:126, 2006; Schmitt et al., Hum. Gene Ther. 20:1240, 2009; June, J. Clin. Invest. 117:1466, 2007) and infectious disease (Kitchen et al., PLoS One 4:38208, 2009; Rossi et al., Nat. Biotechnol. 25:1444, 2007; Zhang et al., PLoS Pathog. 6:e1001018, 2010; Luo et al., J. Mol. Med. 89:903, 2011).

In certain embodiments a hyperproliferative disorder that is a hematological malignancy or a solid cancer is treated. For example, the hematological malignancy to be treated may be acute lymphoblastic leukemia (ALL), acute myeloid leukemia (AML), chronic myelogenous leukemia (CML), chronic eosinophilic leukemia (CEL), myelodysplastic syndrome (MDS), non-Hodgkin's lymphoma (NHL), or multiple myeloma (MM). Exemplary solid cancer to be treated may be biliary cancer, bladder cancer, bone and soft tissue carcinoma, brain tumor, breast cancer, cervical cancer, colon cancer, colorectal adenocarcinoma, colorectal cancer, desmoid tumor, embryonal cancer, endometrial cancer, esophageal cancer, gastric cancer, gastric adenocarcinoma, glioblastoma multiforme, gynecological tumor, head and neck squamous cell carcinoma, hepatic cancer, lung cancer, malignant melanoma, osteosarcoma, ovarian cancer, pancreatic cancer, pancreatic ductal adenocarcinoma, primary astrocytic tumor, primary thyroid cancer, prostate cancer, renal cancer, renal cell carcinoma, rhabdomyosarcoma, skin cancer, soft tissue sarcoma, testicular germ-cell tumor, urothelial cancer, uterine sarcoma, or uterine cancer.

Other exemplary types of cancer that may be treated include adenocarcinoma of the breast, prostate, and colon; all forms of bronchogenic carcinoma of the lung; myeloid leukemia; melanoma; hepatoma; neuroblastoma; papilloma; apudoma; choristoma; branchioma; malignant carcinoid syndrome; carcinoid heart disease; and carcinoma (*e.g*., Walker, basal cell, basosquamous, Brown-Pearce, ductal, Ehrlich tumor, Krebs 2, Merkel cell, mucinous, non-small cell lung, oat cell, papillary, scirrhous, bronchiolar, bronchogenic, squamous cell, and transitional cell). Additional types of cancers that may be treated include histiocytic disorders; malignant histiocytosis; leukemia; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor. Further, the following types of cancers are also contemplated as amenable to treatment: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin. The types of cancers that may be treated also include angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

Exemplifying the variety of hyperproliferative disorders amenable to a fusion protein T cell therapy are B-cell cancers, including B-cell lymphomas (such as various forms of Hodgkin's disease, non-Hodgkins lymphoma (NHL) or central nervous system lymphomas), leukemias (such as acute lymphoblastic leukemia (ALL), chronic lymphocytic leukemia (CLL), Hairy cell leukemia, B cell blast transformation of chronic myeloid leukemia) and myelomas (such as multiple myeloma). Additional B cell cancers include small lymphocytic lymphoma, B-cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt's lymphoma/leukemia, B-cell proliferations of uncertain malignant potential, lymphomatoid granulomatosis, and post-transplant lymphoproliferative disorder.

Inflammatory and autoimmune diseases include arthritis, rheumatoid arthritis, juvenile rheumatoid arthritis, osteoarthritis, polychondritis, psoriatic arthritis, psoriasis, dermatitis, polymyositis/dermatomyositis, inclusion body myositis, inflammatory myositis, toxic epidermal necrolysis, systemic scleroderma and sclerosis, CREST syndrome, inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, systemic lupus erythematosus (SLE), subacute cutaneous lupus erythematosus, discoid lupus, lupus myelitis, lupus cerebritis, juvenile onset diabetes, multiple sclerosis, allergic encephalomyelitis, neuromyelitis optica, rheumatic fever, Sydenham's chorea, immune responses associated with acute and delayed hypersensitivity mediated by cytokines and T-lymphocytes, tuberculosis, sarcoidosis, granulomatosis including Wegener's granulomatosis and Churg-Strauss disease, agranulocytosis, vasculitis (including hypersensitivity vasculitis/angiitis, ANCA and rheumatoid vasculitis), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, myasthenia gravis, antigen-antibody complex mediated diseases, anti-glomerular basement membrane disease, anti-phospholipid antibody syndrome, allergic neuritis, Behcet disease, Castleman's syndrome, Goodpasture's syndrome, Lambert-Eaton Myasthenic Syndrome, Reynaud's syndrome, Sjorgen's syndrome, Stevens-Johnson syndrome, solid organ transplant rejection, graft versus host disease (GVHD), bullous pemphigoid, pemphigus, autoimmune polyendocrinopathies, seronegative spondyloarthropathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), Henoch-Schonlein purpura, autoimmune thrombocytopenia, autoimmune disease of the testis and ovary including autoimmune orchitis and oophoritis, primary hypothyroidism; autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Type I diabetes also referred to as insulin-dependent diabetes mellitus (IDDM) and Sheehan's syndrome; autoimmune hepatitis, lymphoid interstitial pneumonitis (HIV), bronchiolitis obliterans (non-transplant), non-specific interstitial pneumonia (NSIP), Guillain-Barré Syndrome, large vessel vasculitis (including polymyalgia rheumatica and giant cell (Takayasu's) arteritis), medium vessel vasculitis (including Kawasaki's disease and polyarteritis nodosa), polyarteritis nodosa (PAN) ankylosing spondylitis, Berger's disease (IgA nephropathy), rapidly progressive glomerulonephritis, primary biliary cirrhosis, Celiac sprue (gluten enteropathy), cryoglobulinemia, cryoglobulinemia associated with hepatitis, amyotrophic lateral sclerosis (ALS), coronary artery disease, familial Mediterranean fever, microscopic polyangiitis, Cogan's syndrome, Whiskott-Aldrich syndrome and thromboangiitis obliterans.

In particular embodiments, acute myelocytic leukemia, acute lymphocytic leukemia, or chronic myelocytic leukemia is treated.

Infectious diseases include those associated with infectious agents and include any of a variety of bacteria (*e.g*., pathogenic *E. coli, S. typhimurium, P. aeruginosa, B. anthracis, C. botulinum, C. difficile, C. perfringens, H. pylori, V. cholerae, Listeria spp., Rickettsia spp., Chlamydia spp.,* and the like), mycobacteria, and parasites (including any known parasitic member of the Protozoa). Infectious viruses include eukaryotic viruses, such as adenovirus, bunyavirus, herpesvirus, papovavirus, papillomavirus (*e.g*., HPV), paramyxovirus, picornavirus, rhabdovirus (*e.g*., Rabies), orthomyxovirus (*e.g.*, influenza), poxvirus (*e.g.*, Vaccinia), reovirus, retrovirus, lentivirus (*e.g*., HIV), flavivirus (*e.g.*, HCV, HBV) or the like. In certain embodiments, infection with cytosolic pathogens whose antigens are processed and displayed with HLA (MHC) Class I molecules, are treated with fusion proteins of this disclosure.

In a particular embodiment, cells of T cell lineage expressing fusion proteins administered to a subject are syngeneic, allogeneic, or autologous cells.

Pharmaceutical compositions including of the present invention of this disclosure may be administered in a manner appropriate to the disease or condition to be treated (or prevented) as determined by persons skilled in the medical art. An appropriate dose, suitable duration, and frequency of administration of the compositions will be determined by such factors as the condition of the patient, size, type and severity of the disease, particular form of the active ingredient, and the method of administration. The-excipient. Suitable excipients include water, saline, dextrose, glycerol, or the like and combinations thereof.

In some embodiments, the disclosure is directed to a host cell for use in a method v of increasing the activity of an immune cell, enhancing or prolonging an immune response, stimulating an antigen-specific T cell response, inhibiting an immunosuppressive signaling pathway, treating cancer or a tumor, inhibiting immune resistance of cancer cells, or treating an infection, comprising administering to a subject in need thereof an effective amount of a host cell expressing a fusion protein as described herein. In further embodiments, a host cell for use in any of the aforementioned methods further expresses an engineered antigen-specific TCR, an engineered antigen-specific high affinity TCR, a CAR, a co-stimulatory molecule, or any combination thereof. In particular embodiments, methods of treating leukemia are provided, comprising co-expressing a fusion protein as disclosed herein and a recombinant, antigen-specific TCR.

In some embodiments, there are provided methods of inducing or enhancing a Class I HLA response by a CD4+ T cell, comprising administering to a subject in need the host cell of the present invention, wherein thereof an effective amount of the host cell of the present inention, wherein the host cell is a CD4+ T cell.

In any of the aforementioned embodiments, the methods are effective in the absence of administering exogenous IL-2.

In still other embodiments, a subject of any of the aforementioned methods is further treated with an adjunctive therapy, such as a chemotherapy. Exemplary chemotherapeutic agents include, for example, alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSKTM; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel (Taxol^{™}, Bristol-Myers Squibb Oncology, Princeton, N.J.) and docetaxel (Taxotere^{™}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; esperamicins, capecitabine; and pharmaceutically acceptable salts, acids or derivatives of any of the above.

In some embodiments, the adjunctive therapy is a vaccine, an inhibitor of an immunosuppression signal, a B-Raf inhibitor, a MEK inhibitor, a tyrosine kinase inhibitor, a cytotoxic agent, a chemotherapeutic, or any combination thereof. In some embodiments, the inhibitor of an immunosuppression signal is an antibody or siRNA. In some embodiments, the antibody or siRNA is specific for PD-1, PD-L1, PD-L2, CTLA4, LAG3, KIR, CD244, B7-H3, B7-H4, BTLA, HVEM, GAL9, TIM3, A2aR, or any combination thereof.

### EXAMPLES

### EXAMPLE 1

### CYTOKINE AND CHEMOKINE PROFILE IN CARCINOMA CELLS

The soluble factors expressed by purified pancreas carcinoma cells in culture were profiled by quantitative PCR. Briefly, total RNA was extracted (RNeasy Miniprep Kit, Qiagen) from primary cultures of *KPC* tumor epithelial cells and paired metastatic cells to the livers of the same animals (n=3 each), and also from preinvasive pancreatic ductal epithelial cells. RNA was converted to cDNA using a High Capacity Reverse Transcriptase Kit (Applied Biosystems). Quantitative PCR was performed using SYBR Green mastermix and triplicate samples were run on a C1000 Thermal Cycler (BioRad). Primers were based on published literature or designed using Primer-BLAST software. Quantifications were normalized to endogenous *cycA* and fold-change gene expression in invasive and metastatic cells compared to preinvasive cells was calculated using the ΔΔCT method. The genetically-engineered *Kras*^{*LSL-G12D*/+}*. Trp53*^{*LSL-R172H*/+}; *Cre* (*KPC*) mouse model of PDA was previously described by Hingorani et al. (Cancer Cell 7:469, 2005).

The secretory profile of invasive and metastatic cells revealed a substantial commitment to synthesis of growth factors and chemokines involved in both granulocyte (*e.g.*, CXCL1, CXCL2) and monocyte (*e.g.*, CCL2) trafficking (data not shown). Granulocyte macrophage colony stimulating factor (GM-CSF), granulocyte colony stimulating factor (G-CSF), and monocyte colony stimulating factor (M-CSF) were also upregulated in tumor epithelial cells as compared to preinvasive ductal cells. Relative gene expression by quantitative PCR confirmed the increase in these factors in invasive versus preinvasive cells (Figure 1, *see* Stromnes et al., Gut 63:1769, 2014). Of these three myelopoietic cytokines, GM-CSF had the most pronounced effect on promoting granulocytic myeloid-derived suppressor cell (MDSC) survival *in vitro* (data not shown).

As described herein, embodiments provided herein, such as fusion proteins and complexes, are based in some aspect on these and/or related features of tumor microenvironments, such as environments of metastatic or invasive tumors. In some embodiments, provided are compositions and methods to enhance anti-tumor activity of immune therapies (*e.g.,* an exemplary fusion protein comprised of extracellular components from CSF2R (GM-CSFR) and intracellular components from IL-2R is illustrated in Figure 2).

### EXAMPLE 2

### GENERATION OF HIGH AFFINITY MESOTHELIN-SPECIFIC TCRs

B6 Msln^{-/-} and wild-type (WT) mice were immunized with a recombinant adenovirus expressing murine Msln (Ad-Msln) to elicit reactive T cells. T cells specific for epitopes Msln343-351, Msln484-492, Msln544-552, and Msln583-591 were isolated from Msln^{-/-} mice, but not WT mice, consistent with central tolerance (Stromnes et al., Cancer Cell 28:638, 2015). However, both Msln^{-/-} and WT mice generated responses to Msln₄₀₆₋₄₁₄, previously shown to be processed and presented by a B6 ovarian cancer cell line (Hung et al., Gene Ther. 14:921, 2007). Msln₄₀₆₋₄₁₄-specific T cells isolated from WT mice uniformly expressed the Vβ9 TCR chain, as did the majority of Msln₄₀₆₋₄₁₄-specific T cells from Msln^{-/-} mice (Stromnes *et al.,* 2015). Despite expressing similar levels of V09, Msln₄₀₆₋₄₁₄-specific T cell lines from Msln^{-/-}mice stained brighter with tetramer, consistent with higher affinity (Stromnes *et al.,* 2015). Msln^{-/-} Msln₄₀₆₋₄₁₄-specific T cell clones also responded to lower antigen concentrations than the corresponding WT clones (Stromnes *et al.,* 2015). Most T cell clones isolated from WT and Msln^{-/-} mice used the same germline Vα4 and Vβ9 TCR chains, restricting any sequence differences between the highest affinity clones from the respective strains to CDR3 (Figure 3A).

### EXAMPLE 3

### ASSESMENT OF EXEMPLARY CSF2R::IL-2R CONSTRUCTS IN MOUSE MODEL

Exemplary CSF2R::IL-2R chimeric constructs, and impacts thereof on function of antigen-specific engineered T cells, were assessed in a preclinical mouse model for disseminated leukemia, based on the murine C57BL/6 Friend virus-induced erythroleukemia (FBL) and TCR_{gag} transgenic mice.

CSF2R::IL-2R chimeric constructs based on murine genes (similar to those illustrated in Figure 2) and/or a mesothelin-targeted T cell receptor (TCR₁₀₄₅ (MSLN₄₀₆₋₄₁₄ specific))-encoding construct (Stromnes et al., Cancer Cell 28:638, 2015, TCR₁₀₄₅ (including sequence)) were inserted into the pMP71 retroviral vector and used to transduce primary P 14 Thy1.1⁺ mouse splenocytes stimulated with anti-CD3 and anti-CD28 antibodies. Fusion protein constructs were generated by PCR. The constructs were then directionally TOPO-cloned into the pENTR^{™}/D-TOPO^{®} vector (Invitrogen), and transferred into the retroviral vector pMP71-attR using Gateway^{®} technology (Invitrogen). The retroviral packaging cell line Plat-E (Morita et al., 2000, Gene Therapy 7:1063-1066, 2000; Cell Biolabs, Inc.) was transduced with the retroviral vector using effectene transduction reagent (Qiagen). Viral supernatant was collected on days 2 and 3 post-transfection and then used to transduce T cells, in some cases containing TCR₁₀₄₅. One day prior to transfection, P14 Thy1.1⁺ T cells were stimulated with anti-CD3/CD28 and 100 U/mL rhIL-2. Transduction of P14 Thy1.1⁺ T cells was performed in 12 well plates in the presence of IL-2 and polybrene by spinfection for 90 minutes at 1000g. TCR₁₀₄₅ transduced T cells were restimulated with irradiated Thy1.2⁺ splenocytes pulsed with Msln406-414 peptide (GQKMNAQAI, 1 µg/ml) and recombinant human IL-2 (r-IL2, 50 IU/ml) seven days following T cell activation with anti-CD3/CD28. On day 5 after antigen re-stimulation, >90% T cells expressed the introduced TCR. 5 × 10⁶ cells expressing TCR₁₀₄₅ were infused into Thy1.2⁺ C57BL/6 (B6) mice (Jackson Laboratory) together with 5 × 10⁸ pfu of a recombinant attenuated adenovirus vaccine (i.m.) engineered to express recombinant murine mesothelin (Ad-Msln). The infused TCR₁₀₄₅+ donor cells included a population expressing the GMIIL2R fusion protein ("GM/IL2R") and a population not expressing the construct ("WT"). Donor cells were tracked on days 0, 8, 14, and 21 after infusion.

In more detail, TCR₁₀₄₅+ donor (Thy1.1⁺/Vβ9⁺-gated) T cells were analyzed by flow cytometry for surface expression of a molecule containing the extracellular portion of a GM-CSFR (with expression on monocytes used as a positive control). At day 0, approximately 50% of the TCR₁₀₄₅+ donor T cells were observed by this assay to express the chimeric molecule (Figure 3B).

The presence of donor T cells in the blood of the animals continued to be monitored over time. Donor T cells persisted and were detectable in the blood for at least 21 days following transfer (Figure 4A).

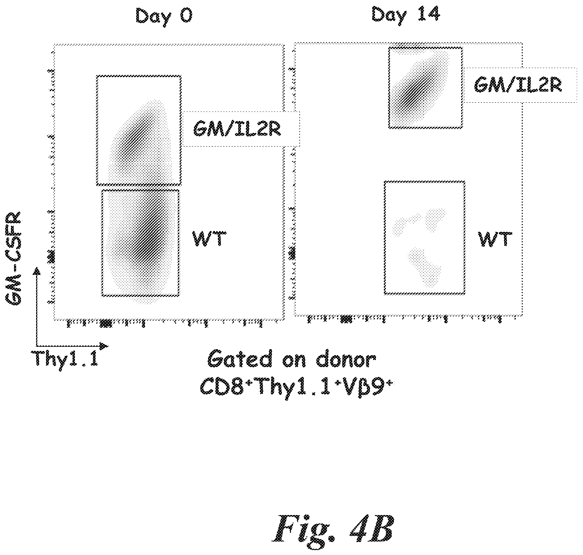

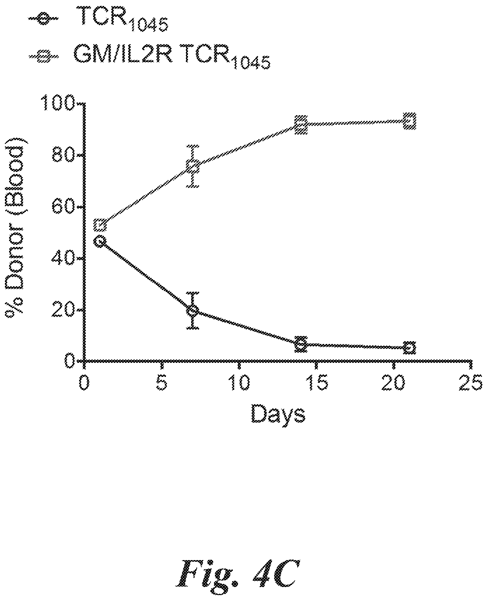

As shown in Figures 4B and 4C, donor T cells expressing the CSF2R::IL-2R fusion protein (as determined by anti-GM-CSF staining) ("GM/IL2R") exhibited a survival and/or proliferative advantage, as compared to donor T cells that did not express the fusion protein ("WT"). For example, by day 14 post-transfer and continuing through day 21, GM/IL2R-expressing cells represented nearly all of the donor T cells detected in the blood (Figures 4B and 4C). These results indicate that the fusion protein provided a persistence advantage to adoptively transferred T cells expressing TCR₁₀₄₅.

These data indicate that fusion proteins of this disclosure in some embodiments provide T cells, such as T cells containing antigen-specific TCRs, a survival and/or expansion advantage, which is consistent with utility of the construct to improve persistence and exposure to transferred cells, including improving efficacy in a tumor microenvironment.

## Claims

1. A host cell, comprising a fusion protein and an antigen binding protein,
wherein the fusion protein comprises a transmembrane domain disposed between an extracellular component comprising a cytokine binding domain or a cytokine binding domain portion thereof, and an intracellular component comprising an IL-2R intracellular portion, intracellular signaling domain or an intracellular signaling domain portion thereof; and
wherein the antigen binding protein is a T cell receptor (TCR), a chimeric antigen receptor (CAR), or both, wherein the TCR is exogenous to the host cell; and
wherein the antigen binding protein is specific for a cancer-specific antigen.

2. The host cell of claim 1, wherein:
the extracellular component comprises an extracellular portion from human CSF2R, CSF1R, CSF3R, CXCR2, or CCR8; or
the cytokine binding domain of the fusion protein specifically binds to human GM-CSF, M-CSF, G-CSF, CXCL1, CXCL2, or CCL1.

3. The host cell of claims 1 or 2, wherein the cytokine binding domain comprises a GM-CSF-specific binding domain, selected from:
an extracellular portion of a CSF2RA comprising an amino acid sequence having at least 90% identity to the amino acid sequence of SEQ ID NO.:9, or
an extracellular portion of a CSF2RB comprising an amino acid sequence having at least 90% identity to the amino acid sequence of SEQ ID NO.: 11.

4. The host cell of any one of the preceding claims, wherein the transmembrane domain of the fusion protein has an amino acid sequence comprising at least 90% identity to the amino acid sequence of SEQ ID NO.:22 or 23, or comprises a transmembrane domain from human IL-2RG, IL-2RB, IL-2RA, IL-4R, IL-7R, IL-9R, IL-15R, IL-21R, CSF2RA, CSF2RB, CSF1R, CSF3R, CXCR2, CCR8, CD2, CD3ε, CD3δ, CD3ζ, CD25, CD27, CD28, CD40, CD79A, CD79B, CD80, CD86, CD95 (Fas), CD134 (OX40), CD137 (4-1BB), CD150 (SLAMF1), CD152 (CTLA4), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR1, ROR2, Ryk, Slp76, SIRPα, pTα, TCRα, TCRβ, TIM3, TRIM, LPA5, or Zap70.

5. The host cell of any one of the preceding claims, wherein the host cell comprises at least two fusion proteins that:
(a) are capable of associating to form a heteromultimer on the host cell surface; and/or
(b) each comprise a different extracellular component and the different extracellular components are capable of associating with each other to form a functional cytokine binding domain; and/or
(c) each comprise a different intracellular component and the different intracellular components are capable of associating with each other to form a functional intracellular signaling domain.

6. The host cell of claim 5, wherein at least one of the different intracellular components comprises: (a) an intracellular signaling domain or an intracellular signaling domain portion thereof from IL-2Rγ, IL-2Rβ, IL-4RA, IL-7R, IL-15RA, or IL-21R, or (b) an amino acid sequence comprising at least 90% identity to the amino acid sequence of SEQ ID NO.: 10, or an amino acid sequence comprising at least 90% identity to the amino acid sequence of SEQ ID NO.: 12.

7. The host cell of claim 5 or 6, wherein:
one of the at least two fusion proteins comprises a transmembrane domain disposed between an extracellular component comprising a cytokine binding domain or a cytokine binding domain portion thereof, and an intracellular component comprised of a functional intracellular signaling domain or an intracellular signaling domain portion thereof from human IL-2Rγ, or has an amino acid sequence comprising at least 90% identity to the amino acid sequence of SEQ ID NO.: 10; and
the other of the at least two fusion proteins comprises a transmembrane domain disposed between an extracellular component comprising a cytokine binding domain or a cytokine binding domain portion thereof, and an intracellular component comprised of: (a) an intracellular signaling domain or an intracellular signaling domain portion thereof from human IL-2Rβ or has an amino acid sequence comprising at least 90% identity to the amino acid sequence of SEQ ID NO.: 12, and/or (b) a signaling domain or a signaling domain portion thereof from IL-4R, IL-7R, IL-9R, IL-15R or IL-21R.

8. The host cell of any one of claims 5-7, wherein the heteromultimer on the host cell surface is a heterodimer or heterotrimer.

9. The host cell of claim 8, wherein:
(A) one of the at least two fusion proteins:
(i) has an amino acid sequence comprising at least 90% identity to the amino acid sequence of SEQ ID NO.: 1;
(ii) comprises the amino acid sequence of SEQ ID NO.: 1; or
(iii) consists of the amino acid sequence of SEQ ID NO.:1; and
(B) the other of the at least two fusion proteins:
(i) has and amino acid sequence comprising at least 90% identity to the amino acid sequence of SEQ ID NO.:2;
(ii) comprises the amino acid sequence of SEQ ID NO.:2; or
(iii) consists of the amino acid sequence of SEQ ID NO.:2.

10. The host cell of any one of the preceding claims, wherein the exogenous TCR binds to an antigen::HLA complex at a Kₐ equal to or greater than 10⁷ M⁻¹.

11. The host cell of any one of the preceding claims, wherein the cancer-specific antigen comprises WT-1, mesothelin, ROR1, or cyclin-A1 antigen.

12. The host cell of any one of the preceding claims, wherein the expression of the fusion protein in a T cell comprising a TCR or chimeric antigen receptor specific for the cancer-specific antigen results in at least about a 1.5-fold, 2-fold, or 3-fold increase in survival, expansion, cytotoxicity, cytokine secretion, and/or response to multiple rounds of stimulation, by the T cell, in response to binding of the antigen and/or following administration to a subject, and/or results in at least about a 1.5-fold, 2-fold, or 3-fold increase in time of survival, disease-free survival, or amelioration of one or more disease symptom, of a subject to which the cell is administered, as compared to a cell substantially the same as the T cell but not containing the fusion protein.

13. A host cell, comprising a nucleic acid molecule encoding the fusion protein and the antigen binding protein of any one of claims 1-12.

14. A composition comprising the host cell of any one of the preceding claims and a pharmaceutically acceptable carrier, diluent, or excipient.

15. The host cell according to any one of claims 1-13, or the composition according to claim 14 for use in treating cancer.

16. The host cell or composition for use of claim 15, wherein the cancer is a tumor selected from:
a hematologic tumor, which is optionally a hematologic tumor selected from CLL or MCL, or
a solid tumor, which optionally is a solid tumor selected from a breast cancer, lung cancer, ovarian cancer, or pancreatic cancer tumor, or which optionally is a solid tumor selected from a lung adenocarcinoma, adenocarcinoma, squamous cell carcinoma, small cell carcinoma, atypical carcinoid, or triple-negative breast cancer.

## Patentansprüche

1. Wirtszelle umfassend ein Fusionsprotein und ein antigenbindendes Protein, wobei das Fusionsprotein eine Transmembrandomäne umfasst, die zwischen einer extrazellulären Komponente, umfassend eine Zytokin-bindende Domäne oder einen Zytokin-bindenden Domänenteil davon, und einer intrazellulären Komponente, umfassend einen IL-2R intrazellulären Teil, intrazelluläre Signaldomäne oder einen intrazellulären Signaldomänenteil davon, angeordnet ist; und wobei das antigenbindende Protein ein T-Zell-Rezeptor (TCR), ein chimärer Antigenrezeptor (CAR), oder beides ist, wobei der TCR exogen zu der Wirtszelle ist; und
wobei das antigenbindende Protein spezifisch für ein krebsspezifisches Antigen ist.

2. Wirtszelle nach Anspruch 1, wobei:
die extrazelluläre Komponente einen extrazellulären Teil des menschlichen CSF2R, CSF1R, CSF3R, CXCR2, oder CCR8 umfasst; oder die Zytokin-bindende Domäne des Fusionsproteins spezifisch an das menschliche GM-CSF, M-CSF, G-CSF, CXCL1, CXCL2, oder CCL1 bindet.

3. Wirtszelle nach Ansprüchen 1 oder 2, wobei die Zytokin-bindende Domäne eine GM-CSF-spezifische Bindungsdomäne umfasst, ausgewählt aus:
einem extrazellulären Teil eines CSF2RA, der eine Aminosäuresequenz mit mindestens 90% Identität mit der Aminosäuresequenz von SEQ ID NO.: 9 umfasst, oder
einem extrazellulären Teil eines CSF2RB, der eine Aminosäuresequenz mit mindestens 90% Identität mit der Aminosäuresequenz von SEQ ID No.: 11 umfasst.

4. Wirtszelle nach irgendeinem der vorhergehenden Ansprüche, wobei die Transmembrandomäne des Fusionsproteins eine Aminosäuresequenz, die mindestens 90% Identität mit der Aminosäuresequenz von SEQ ID NO.:22 oder 23 umfasst, hat, oder eine Transmembrandomäne des menschlichen IL-2RG, IL-2RB, IL-2RA, IL-4R, IL-7R, IL-9R, IL-15R, IL-21R, CSF2RA, CSF2RB, CSF1R, CSF3R, CXCR2, CCR8, CD2, CD3ε, CD3δ, CD3ζ, CD25, CD27, CD28, CD40, CD79A, CD79B, CD80, CD86, CD95 (Fas), CD134 (OX40), CD137 (4-1BB), CD150 (SLAMF1), CD152 (CTLA4), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FeRγ, Fyn, GAL9, KIR, Lck, LAT, LRP, NKG2D, NOTCH1, NOTCH2, NOTCH3, NOTCH4, PTCH2, ROR1, ROR2, Ryk, Slp76, SIRPα, pTα, TCRα, TCRβ, TIM3, TRIM, LPA5, oder Zap70 umfasst.

5. Wirtszelle nach irgendeinem der vorhergehenden Ansprüche, wobei die Wirtszelle mindestens zwei Fusionsproteine umfasst, die:
(a) in der Lage sind, sich zu assoziieren, um einen Heteromultimer auf der Oberfläche der Wirtszelle zu bilden; und/oder
(b)jeweils eine unterschiedliche extrazelluläre Komponente umfassen und die unterschiedlichen extrazellulären Komponenten in der Lage sind, sich miteinander zu assoziieren, um eine funktionale Zytokin-bindende Domäne zu bilden; und/oder
(c) jeweils eine unterschiedliche intrazelluläre Komponente umfassen und die unterschiedlichen intrazellulären Komponenten in der Lage sind, sich miteinander zu assoziieren, um eine funktionale intrazelluläre Signaldomäne zu bilden.

6. Wirtszelle nach Anspruch 5, wobei mindestens eine der unterschiedlichen intrazellulären Komponenten umfasst:
(a) eine intrazelluläre Signaldomäne oder einen intrazellulären Signaldomänenteil davon des IL-2Rγ, IL-2Rβ, IL-4RA, IL-7R, IL-15RA, oder IL-21R, oder (b) eine Aminosäuresequenz, die mindestens 90% Identität mit der Aminosäuresequenz von SEQ ID NO.: 10 umfasst, oder eine Aminosäuresequenz, die mindestens 90% Identität mit der Aminosäuresequenz von SEQ ID NO.: 12 umfasst.

7. Wirtszelle von Anspruch 5 oder 6, wobei:
eines der mindestens zwei Fusionsproteine eine Transmembrandomäne umfasst, die zwischen einer extrazellulären Komponente, umfassend eine Zytokin-bindende Domäne oder einen Zytokin-bindenden Domänenteilteil davon, und einer intrazellulären Komponente, umfassend eine funktionale intrazelluläre Signaldomäne oder einen intrazellulären Signaldomänenteil davon des menschlichen IL-2Rγ, angeordnet ist, oder eine Aminosäuresequenz, umfassend mindestens 90% Identität mit der Aminosäuresequenz von SEQ ID NO.: 10, hat; und das andere der mindestens zwei Fusionsproteine eine Transmembrandomäne umfasst, die zwischen einer extrazellulären Komponente, die eine Zytokin-bindenden Domäne oder einen Zytokin-bindenden Domänenteil davon umfasst, und einer intrazellulären Komponente, die: (a) eine intrazelluläre Signaldomäne oder einen intrazellulären Signaldomänenteil davon des menschlichen IL-2Rβ umfasst, oder eine Aminosäuresequenz, umfassend mindestens 90% Identität mit der Aminosäuresequenz von SEQ ID NO.: 12, hat und/oder (b) eine Signaldomäne oder einen Signaldomänenteil davon des IL-4R, IL-7R, IL-9R, IL15R oder IL-21R umfasst, angeordnet ist.

8. Wirtszelle nach irgendeinem der Ansprüche 5-7, wobei der Heteromultimer auf der Oberfläche der Wirtszelle ein Heterodimer oder Heterotrimer ist.

9. Wirtszelle nach Anspruch 8, wobei:
(A) eines der mindestens zwei Fusionsproteine:
(i) eine Aminosäuresequenz, umfassend mindestens 90% Identität mit der Aminosäuresequenz von SEQ ID NO.:1, hat;
(ii) die Aminosäuresequenz von SEQ ID NO.:1 umfasst; oder
(iii) aus der Aminosäuresequenz von SEQ ID NO.:1 besteht; und
(B) das andere der mindestens zwei Fusionsproteine:
(i) eine Aminosäuresequenz, umfassend mindestens 90% Identität mit der Aminosäuresequenz von SEQ ID NO.:2, hat;
(ii) die Aminosäuresequenz von SEQ ID NO.:2 umfasst; oder
(iii) aus der Aminosäuresequenz von SEQ ID NO.:2 besteht.

10. Wirtszelle nach irgendeinem der vorhergehenden Ansprüche, wobei der exogene TCR an einen Antigen::HLA Komplex mit einem Kₐ gleich oder größer als 10⁷ M⁻¹ bindet.

11. Wirtszelle nach irgendeinem der vorhergehenden Ansprüche, wobei das krebsspezifische Antigen WT-1, Mesothelin, ROR1, oder Cyclin-A1 Antigen umfasst.

12. Wirtszelle nach irgendeinem der vorhergehenden Ansprüche, wobei die Expression des Fusionsproteins in einer T-Zelle, die einen TCR oder einen für das krebsspezifische Antigen spezifischen chimären Antigenrezeptor umfasst, zu einer mindestens etwa 1,5-fachen, 2-fachen, oder 3-fachen Steigerung der Überlebensrate, Expansion, Zytotoxizität, Zytokin-Ausschüttung und/oder Antwort auf mehrere Stimulationsrunden, durch die T-Zelle, als Antwort auf Bindung an das Antigen und/oder nach Verabreichung an ein Subjekt führt und/oder zu einer mindestens etwa 1,5-fachen, 2-fachen, oder 3-fachen Steigerung der Überlebenszeit, des krankheitsfreien Überlebens oder der Verbesserung von einem oder mehreren Krankheitssymptomen eines Subjekts führt, dem die Zelle verabreicht wurde, im Vergleich zu einer Zelle, die im Wesentlichen die gleiche T-Zelle ist, außer dass sie das Fusionsprotein nicht beinhaltet.

13. Wirtszelle umfassend ein Nukleinsäuremolekül, das das Fusionsprotein und das antigenbindende Protein nach irgendeinem der Ansprüche 1-12 kodiert.

14. Zusammensetzung umfassend die Wirtszelle nach irgendeinem der vorhergehenden Ansprüche und einen pharmazeutisch annehmbaren Träger, Verdünnungsmittel oder Hilfsstoff.

15. Wirtzelle gemäß irgendeinem der Ansprüche 1-13 oder die Zusammensetzung gemäß Anspruch 14 zur Verwendung bei der Behandlung von Krebs.

16. Wirtszelle oder Zusammensetzung zur Verwendung nach Anspruch 15, wobei der Krebs ein Tumor ist, ausgewählt aus: einem hämatologischen Tumor, der optional ein hämatologischer Tumor ist, ausgewählt aus CLL oder MCL, oder einem festen Tumor, der optional ein fester Tumor ist, ausgewählt aus Brustkrebs-, Lungenkrebs-, Eierstockkrebs-, oder Bauchspeicheldrüsenkrebstumor, oder, der optional ein fester Tumor ist, ausgewählt aus Lungenadenokarzinom, Adenokarzinom, Plattenepithelkarzinom, kleinzelligem Karzinom, atypischem Karzinoid, oder triple-negativem Brustkrebs.

## Revendications

1. Cellule hôte, comprenant une protéine de fusion et une protéine de liaison à l'antigène,
dans laquelle la protéine de fusion comprend un domaine transmembranaire disposé entre un composant extracellulaire comprenant un domaine de liaison de cytokine ou une partie de domaine de liaison de cytokine de celui-ci, et un composant intracellulaire comprenant une partie intracellulaire IL-2R, un domaine de signalisation intracellulaire ou une partie de domaine de signalisation intracellulaire de celui-ci ; et
dans laquelle la protéine de liaison à l'antigène est un récepteur de cellules T (TCR), un récepteur antigénique chimérique (CAR), ou les deux, dans laquelle le TCR est exogène à la cellule hôte ; et
dans laquelle la protéine de liaison à l'antigène est spécifique pour un antigène spécifique d'un cancer.

2. Cellule hôte selon la revendication 1, dans laquelle :
le composant extracellulaire comprend une partie extracellulaire provenant de CSF2R, CSF1R, CSF3R, CXCR2, ou CCR8 humain ; ou
le domaine de fusion de cytokine de la protéine de fusion se lie spécifiquement à GM-CSF, M-CSF, G-CSF, CXCL1, CXCL2, ou CCL1 humain.

3. Cellule hôte selon la revendication 1 ou la revendication 2, dans laquelle le domaine de liaison de cytokine comprend un domaine de liaison spécifique de GM-CSF, sélectionné à partir :
d'une protéine extracellulaire d'un CSF2RA comprenant une séquence d'acides aminés présentant une identité d'au moins 90 % de la séquence d'acides aminés de SEQ ID NO : 9, ou
d'une partie extracellulaire d'un CSF2RB comprenant une séquence d'acides aminés présentant une identité d'au moins 90 % de la séquence d'acides aminés de SEQ ID NO : 11.

4. Cellule hôte selon l'une quelconque des revendications précédentes, dans laquelle le domaine transmembranaire de la protéine de fusion présente une séquence d'acides aminés comprenant une identité d'au moins 90 % de la séquence d'acides aminés de SEQ ID NO : 22 ou 23, ou comprend un domaine transmembranaire à partir de IL-2RG, IL-2RB, IL-2RA, IL-4R, IL-7R, IL-9R, IL-15R, IL-21R, CSF2RA, CSF2RB, CSF1R, CSF3R, CXCR2, CCR8, CD2, CD3ε, CD3δ, CD3ζ, CD25, CD27, CD28, CD40, CD79A, CD79B, CD80, CD86, CD95 (Fas), CD134 (OX40), CD137 (4-1BB), CD150 (SLAMF1), CD152 (CTLA4), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR1, ROR2, Ryk, Slp76, SIRPα, pTα, TCRα, TCRβ, TIM3, TRIM, LPA5, ou Zap70 humain.

5. Cellule hôte selon l'une quelconque des revendications précédentes, dans laquelle la cellule hôte comprend au moins deux protéines de fusion qui :
(a) sont capables de s'associer pour former un hétéro-multimère sur la surface de cellule hôte ; et/ou
(b) comprennent chacune un composant extracellulaire différent et les différents composants extracellulaires sont capables de s'associer les uns aux autres pour former un domaine de liaison de cytokine fonctionnel ; et/ou
(c) comprennent chacune un composant intracellulaire différent et les différents composants intracellulaires sont capables de s'associer les uns aux autres pour former un domaine de signalisation intracellulaire fonctionnel.

6. Cellule hôte selon la revendication 5, dans laquelle au moins un des différents composants intracellulaires comprend : (a) un domaine de signalisation intracellulaire ou une partie de domaine de signalisation intracellulaire de celui-ci à partir de IL-2Rγ, IL-2Rβ, IL-4RA, IL-7R, IL-15RA, ou IL-21R, ou (b) une séquence d'acides aminés comprenant une identité d'au moins 90 % de la séquence d'acides aminés de SEQ ID NO : 10, ou une séquence d'acides aminés comprenant une identité d'au moins 90 % de la séquence d'acides aminés de SEQ ID NO : 12.

7. Cellule hôte selon la revendication 5 ou revendication 6, dans laquelle :
une des au moins deux protéines de fusion comprend un domaine transmembranaire disposé entre un composant extracellulaire comprenant un domaine de liaison de cytokine ou une partie de domaine de liaison de cytokine de celui-ci, et un composant intracellulaire composé d'un domaine de signalisation intracellulaire fonctionnel ou d'une partie de domaine de signalisation intracellulaire de celui-ci à partir de IL-2Rγ humain, ou présente une séquence d'acides aminés comprenant une identité d'au moins 90 % de la séquence d'acides aminés de SEQ ID NO : 10 ; et
l'autre des au moins deux protéines de fusion comprend un domaine transmembranaire disposé entre un composant extracellulaire comprenant un domaine de liaison de cytokine ou une partie de domaine de liaison de cytokine de celui-ci, et un composant intracellulaire composé de : (a) un domaine de signalisation intracellulaire ou une partie de domaine de signalisation intracellulaire de celui-ci à partir de IL-2Rβ humain ou présente une séquence d'acides aminés comprenant une identité d'au moins 90 % de la séquence d'acides aminés de SEQ ID NO : 12, et/ou (b) un domaine de signalisation ou une partie de domaine de signalisation de celui-ci à partir de IL-4R, IL-7R, IL-9R, IL-15R ou IL-21R.

8. Cellule hôte selon l'une quelconque des revendications 5 à 7, dans laquelle l'hétéro-multimère sur la surface de cellule hôte est un hétérodimère ou hétérotrimère.

9. Cellule hôte selon la revendication 8, dans laquelle :
(A) une des au moins deux protéines de fusion :
(i) présente une séquence d'acides aminés comprenant une identité d'au moins 90 % de la séquence d'acides aminés de SEQ ID NO : 1 ;
(ii) comprend la séquence d'acides aminés de SEQ ID NO : 1 ; ou
(iii) est constituée de la séquence d'acides aminés de SEQ ID NO : 1 ; et
(B) l'autre des au moins deux protéines de fusion :
(i) présente une séquence d'acides aminés comprenant une identité d'au moins 90 % de la séquence d'acides aminés de SEQ ID NO : 2 ;
(ii) comprend la séquence d'acides aminés de SEQ ID NO : 2 ; ou
(iii) est constituée de la séquence d'acides aminés de SEQ ID NO : 2.

10. Cellule hôte selon l'une quelconque des revendications précédentes, dans laquelle le TCR exogène se lie à un complexe antigène::HLA à un Kₐ égal ou supérieur à 10⁷ M⁻¹.

11. Cellule hôte selon l'une quelconque des revendications précédentes, dans laquelle l'antigène spécifique d'un cancer comprend un antigène WT-1, mésothéline, ROR1 ou cycline-A1.

12. Cellule hôte selon l'une quelconque des revendications précédentes, dans laquelle l'expression de la protéine de fusion est une cellule T comprenant un TCR ou récepteur antigénique chimérique spécifique pour l'antigène spécifique d'un cancer résulte en une augmentation d'au moins environ 1,5 fois, 2 fois ou 3 fois de la survie, de l'expansion, de la cytotoxicité, de la sécrétion de cytokine, et/ou de la réponse à de multiples cycles de stimulation, par la cellule T, en réponse à la liaison de l'antigène et/ou suivant l'administration chez un sujet, et/ou résulte en une augmentation d'au moins environ 1,5 fois, 2 fois ou 3 fois du temps de survie, de survie sans maladie, ou de l'amélioration d'un ou de plusieurs symptômes de maladie, d'un sujet auquel la cellule est administrée, par rapport à une cellule qui est sensiblement la même que la cellule T, mais qui ne contient pas la protéine de fusion.

13. Cellule hôte, comprenant une molécule d'acide nucléique codant la protéine de fusion et la protéine de liaison à l'antigène selon l'une quelconque des revendications 1 à 12.

14. Composition comprenant la cellule hôte selon l'une quelconque des revendications précédentes et un milieu, diluant ou excipient de qualité pharmaceutique.

15. Cellule hôte selon l'une quelconque des revendications 1 à 13, ou composition selon la revendication 14 destinée à être utilisée dans le traitement d'un cancer.

16. Cellule hôte ou composition destinée à être utilisée selon la revendication 15, dans laquelle le cancer est une tumeur sélectionnée parmi :
une tumeur hématologique, qui est éventuellement une tumeur hématologique sélectionnée à partir de CLL ou de MCL, ou
une tumeur solide, qui est éventuellement une tumeur solide sélectionnée parmi une tumeur de cancer du sein, de cancer du poumon, de cancer ovarien, ou de cancer du pancréas, ou qui est éventuellement une tumeur solide sélectionnée parmi un adénocarcinome du poumon, un adécocarcinome, un carcinome de cellule squameuse, un carcinome à petites cellules, un carcinoïde atypique, ou cancer du sein triple négatif.
